# EUROPEAN PATENT APPLICATION

(11) **EP 1 860 113 A1**
(43) Date of publication of application: **28.11.2007**
(21) Application number: 06114534.8
(22) Date of filing: 24.05.2006
(51) Int. Cl.: C07H 19/06, C07H 19/16, A61P 29/00, A61P 27/02, A61P 25/00, A61P 11/00, A61K 31/7052

(54) **Ectonucleotidase inhibitors**

(71) Applicant: Rheinische Friedrich-Wilhelms-Universität Bonn, 53113 Bonn (DE)
(72) Inventor: Müller, Christa E., 53113, Bonn (DE); Brunschweiger, Andreas, 53225, Bonn-Beuel (DE); Igbal, Jamshed, 53115, Bonn (DE)
(74) Representative: Helbing, Jörg

(57) **Abstract**

Present invention provides ectonucleotidase (ecto-nucleotide triphosphate diphosphohydrolase, NTPDase) inhibitors represented by the following formula, namely nucleotide mimetics as selective NTPDase inhibitors. It also provides methods for preparations of said compounds. Furthermore provided are pharmaceutical and diagnostic compositions comprising said compounds, and the use of said compounds in a medicament for treating diseases associated with ectonucleotidase activity and/or P2 receptors.

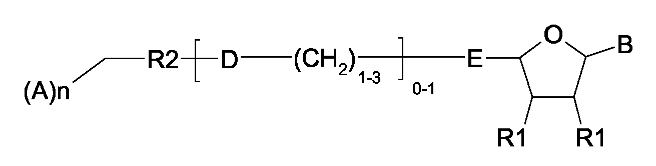

## Description

Present invention provides ectonucleotidase (ecto-nucleotide triphosphate diphosphohydrolase, NTPDase) inhibitors, namely nucleotide mimetics as selective NTPDase inhibitors. It also provides methods for preparations of said compounds. Furthermore provided are pharmaceutical and diagnostic compositions comprising said compounds, and the use of said compounds in a medicament for treating diseases associated with ectonucleotidase activity and/or P2 receptors.

### Background of the Invention

Extracellular nucleotides such as ATP, ADP, UTP, and UDP can act as activators/agonists on a variety of nucleotide receptors (P2 receptors), namely purine P2 receptors and/or pyrimidine P2 receptors (Ralevic, V., and Burnstock, G., Pharmacol Rev 1998; 50: 413-92). The activation of P2 receptors is controlled by ecto-nucleotidases (NTPDases) capable of hydrolyzing nucleoside tri- and diphosphates (Zimmermann, H., Naunyn Schmiedebergs Arch Pharmacol 2000; 362: 299-309). Inhibition of ecto-nucleotidases can result in a potentiation of purinergic signaling, supporting the notion that endogenous ecto-nucleotidases reduce the effective concentration of the released nucleotide (Crack, B.E. et al., Br J Pharmacol 1994; 113: 1432-8; Crack, B.E. et al., Br J Pharmacol 1995; 114: 475-81; Bültmann, R. et al., Naunyn Schmiedebergs Arch Pharmacol 1995; 351: 555-60). Similarly, metabolically stable analogs of ATP are considerably more effective in causing a biological response than ATP itself (for references see Zimmermann, H., Ecto-nucleotidases. In Abbracchio, M.P. and Williams, M. (eds): Handbook of Experimental Pharmacology. Purinergic and Pyrimidergic Signalling, Heidelberg: Springer Verlag 2001; 209-50). Inhibitors of ectonucleotidases could thus represent valuable tools for amplifying the biological effects induced by extracellularly released nucleotides. In addition, inhibition of ecto-nucleotidases is mandatory for both, studies of nucleotide release and the analysis of the potency on P2 receptors of nucleotides or their hydrolyzable analogs. P2 receptors are divided in two categories: G protein-coupled receptors, termed P2Y (currently known subtypes: P2Y₁, P2Y₂, P2Y₄, P2Y₆, P2Y₁₁, P2Y₁₂, P2Y₁₃, P2Y₁₄) and ligand-gated cation channels, termed P2X (currently known subtypes: P2X₁₋₇). Several subtypes have been cloned within each family, and function in such systems as the central and peripheral nervous systems, the cardiovascular system, the endocrine system, lung, intestines, muscle, and the immune system (Xu, B. et al., J. Med. Chem. 2002; 45: 5694-5709; Fredholm, B.B. et al., Trends Pharm. Sci. 1997; 18: 79-82; Di Virgilio, F. et al., Blood 2001; 97: 587-600; Burnstock, G. and Williams, M., J. Pharmacol. Exp. Ther. 2000; 295: 862-869).

Inhibitors of ecto-nucleotidases should have no effect on P2 receptors and should not be dephosphorylated by ecto-nucleotidase. Ideally they would also reveal selectivity for individual NTPDase isoforms. Many antagonists of P2 receptors also act as inhibitors of ecto-nucleotidases. These include suramin, pyridoxalphosphate-6-azophenyl-2',4'-disulfonic acid (PPADS) and reactive blue 2 (for references see Zimmermann, H., Ecto-nucleotidases. In Abbracchio, M.P. and Williams, M. (eds): Handbook of Experimental Pharmacology. Purinergic and Pyrimidergic Signalling, Heidelberg: Springer Verlag 2001; 209-50): NTPDase 2, for example, is predominantly expressed by hippocampal, cortical and cerebellar astrocytes. The enzyme probably modulates inflammatory reactions in the CNS and may therefore represent a useful therapeutic target in human diseases.

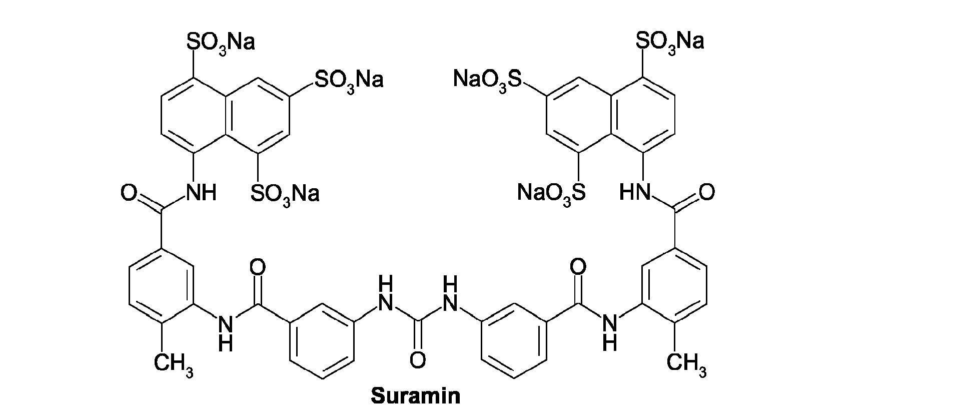

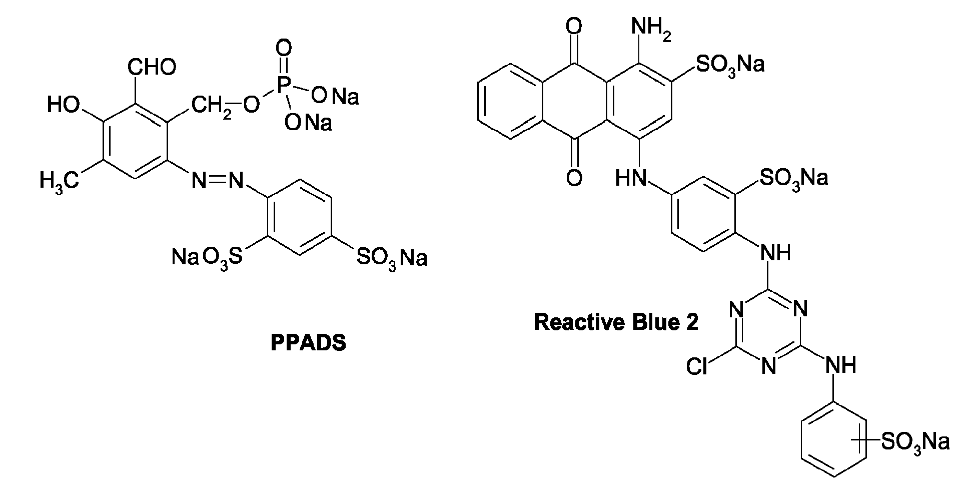

To date only the ATP analog ARL67156 (FPL67156, *N⁶*-Diethyl-β,γ-dibromomethylene-ATP) (Crack, B.E. et al., Br J Pharmacol 1995; 114: 475-81; Kennedy, C. et al., Semin Neurosci 1996; 8: 195-99) 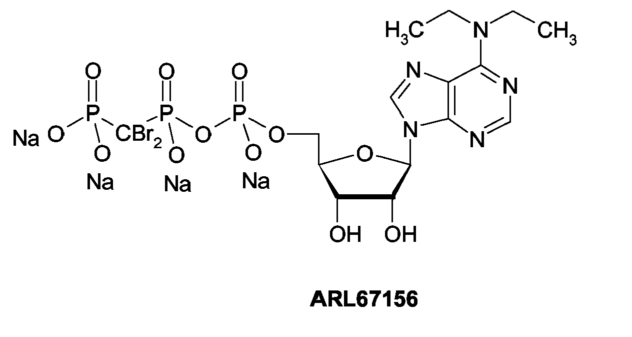 and 8-thiobutyladenosine 5' -triphosphate (8-Bu-S-ATP) (Gendron, F.P. et al., J Med Chem 2000; 43: 2239-47) reveal enzyme inhibitory potential without significantly affecting nucleotide receptors. However, these compounds are not very potent, they are highly polar, and - since they contain phosphoric acid ester bonds - will probably not be highly stable but be hydrolyzed by physiological enzymes.

The ecto-nucleoside triphosphate diphosphohydrolases (EC 3.6.1.5) represent a major and ubiquitous family of ecto-nucleotidases. They catalyze the sequential hydrolysis of the γ- and β-phosphate residues of nucleoside tri- and diphosphates, producing the corresponding nucleoside monophosphate derivatives (Zimmermann, H., Naunyn Schmiedebergs Arch Pharmacol 2000; 362: 299-309). To date four different cell surface-located isoforms of the enzyme family have been cloned and functionally characterized (NTPDase1, 2 and 3, and very recently NTPDase8 (Bigonnesse, F. et al., Biochemistry 2004; 43: 5511-9; Zimmermann, H., Drug Dev Res 2001; 52: 44-56; Kukulski, F. et al., Purinergic Signalling 2005; 1: 193-204). The four enzymes differ in substrate specificity and in the pattern of product formation. Whereas NTPDase1 hydrolyzes ATP and

ADP about equally well, NTPDase2 has a high preference for the hydrolysis of ATP over ADP. NTPDase3 and NTPDase8 are functional intermediates. NTPDase1 hydrolyzes ATP directly to AMP, ADP is the preferential product of ATP hydrolysis by NTPDase2, and NTPDase3 and NTPDase8 hydrolyze ADP formed from ATP efficiently to AMP. The different isoenzymes show distinct expression profiles.

Potential therapeutic applications of NTPDase inhibitors include all disease therapies which aim at increasing the nucleotid concentration in a patient (Ralevic, V., and Burnstock, G., Pharmacol Rev 1998; 50: 413-92; Brunschweiger, A. and Müller, C.E., Curr. Med. Chem. 2006, 13, 289-312; Vekaria, R. M. et al., Am. J Physiol Renal Physiol 2006, 290, F550-F560; Gendron, F.P. et al., Curr Drug Targets 2002, 3, 229-245). Potential applications include dry eye disease (local application), respiratory diseases, cystic fibrosis, inflammatory diseases, diseases of the immune system, gastrointestinal diseases, kidney disorders, cancer, and brain diseases. Furthermore, selective NTPDase inhibitors may be useful for diagnostic purposes and as pharmacological tools.

For example, NTPDase2 is predominantly expressed by hippocampal, cortical and cerebellar astrocytes. The enzyme probably modulates inflammatory reactions in the CNS and therefore represents a potential therapeutical target (Wish, M.R. et al., Neuroscience 2006, in press).

However, so far no highly potent and at the same time highly selective inhibitors for certain subtypes of NTPDases have been described. Such compounds could increase extracellular nucleotide concentrations in an event- and site-specific manner and thus act as indirect, "selective" P2 receptor agonists. Selective NTPDase inhibitors should not exhibit affinity for P2 receptors. NTPDase inhibitors showing the desired properties may be used as novel therapeutics (drugs) for various diseases.

Up to now it has mainly been attempted to develop direct P2 receptor agonists. Major drawbacks of such compounds are (i) that they do not act in a site- and event-specific manner, but at all receptors, and not only where the nucleotide concentration is high; (ii) the P2 agonists that are known so far are highly polar containing negative charges, since they are derived from nucleotides. Therefore they are only parenterally applicable.

Thus, the problem underlying present invention is the provision of NTPDase inhibitors which are not highly polar, do not block P2 receptors and which preferably act in a site and event specific manner.

### Summary of the invention

Present invention provides new NTPDase inhibitors which are not nucleotides, but nucleotide mimetics. Preferably, said compounds are neutral (not anionic/negatively charged). The compounds are selective versus P2 receptors. They exhibit high potency to inhibit NTPDases and some are selective for certain NTPDase subtypes. The new compounds are derivatives of nucleosides or nucleoside derivatives; they can be described as nucleotide mimetics, in which the phosphate chain of the corresponding nucleotides is replaced by various substituents of different lengths, preferably bearing a terminal phosphonic acid diester group. The nucleobase can be derivatized or otherwise modified. The ribose moiety can also be modified. Such compounds should show peroral bioavailability. In contrast to nucleotides, the new compounds are metabolically considerably more stable. The new compounds are competitive inhibitors of NTPDases. They are suitable for the treatment of a number of different diseases in which the activation of P2 receptors is advantageous.

Thus, the present invention provides
(1) a compound represented by the formula 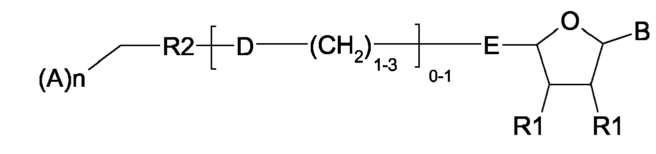 wherein
   D represents a moiety selected from the group consisting of a single bond, -O-, -S-, -CH₂-, -CHR3-, -NH-, -NR3-,-CO-, -CH₂CO-, 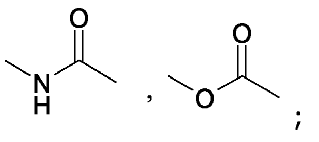
   E represents a moiety selected from the group consisting of -R5-, -O-R5-, -SCH₂- and -NH-R5-;
   B represents a purinyl or pyrimidinyl residue which is connected with the furanoside ring via one of its nitrogen atoms;
   R1 represent independently from each other residues selected from the group consisting of hydroxyl, hydrogen, C₁-C₃-alkoxyl, C₁-C₃-alkyl, C₁-C₃-alkenyl, C₁-C₃-alkinyl, C₁-C₃-acyl, halogen, or commonly form a double bond with one of the vicinal C atoms or an acetyl or ketal ring with each other;
   R2 is -(CH₂)₀₋₂- or phenyliden;
   n is 1 or 2;
   A represents a -PO(OR3)₂, -SO2(OR3), or -(CH₂)ₘ-COOR4 residue, wherein m is an integer from 0 to 2, R3 is a C₁-C₃-alkyl and R4 is selected from the group consisting of hydrogen and C₁₋C₃-alkyl; and
   R5 is a carbonyl or methylidene group;
(2) a pharmaceutical or diagnostic composition comprising a compound as defined in (1) above;
(3) the use of the compound as defined in (1) above for the preparation of a medicament for treating diseases connected with a reduced abundance of nucleotides in a patient or for therapies aiming at increasing the nucleotide concentration in a patient;
(4) the use of the compound as defined in (1) above as selective NTPDase inhibitor;
(5) an *in vitro* method for ATP quantification using the compound as defined in (1) above; and
(6) a method for preparing the compound as defined in (1) above.

### Detailed Description of Invention

The compounds of present invention are structurally derived from nucleosides. I their broadest sense, they can be seen as nucleotide-mimetics wherein the phosphate chain is replaced with moieties which are less prone to hydrolysis.

In a preferred aspect of said mimetics, the phosphate chain is replaced by a carbohydrate chain forming an amide or amine with the ribose on one end and bearing an ester or acid group on the other end. Thus, this preferred compound is represented by the following formula (I): 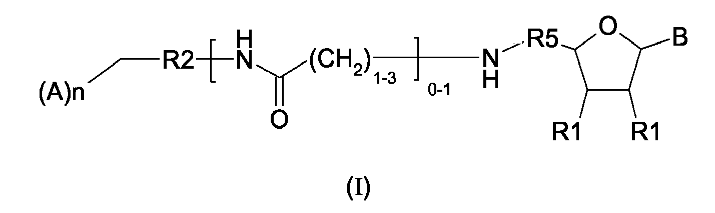 wherein
B represents a purinyl or pyrimidinyl residue which is connected with the furanoside ring via one of its nitrogen atoms;
R1 represent independently from each other residues selected from the group consisting of hydroxyl, hydrogen, C₁-C₃-alkoxyl, C₁-C₃-alkyl, C₁-C₃-alkenyl, C₁-C₃-alkinyl, C₁-C₃-acyl, halogen, or commonly form a double bond with one of the vicinal C atoms or an acetyl or ketal ring with each other;
R2 is -(CH₂)₀₋₂- or phenyliden;
n is 1 or 2;
A represents a -PO(OR3)₂, -SO2(OR3), or -(CH₂)ₘ-COOR4 residue, wherein m is an integer from 0 to 2, R3 is a C₁-C₃-alkyl and R4 is selected from the group consisting of hydrogen and C₁-C₃-alkyl; and
R5 is a carbonyl or methylidene group.

In more detail, in the formula representing the compound of embodiment (1) and in the preferred formula (I), the variables are defined as follows:
B represents a purinyl or pyrimidinyl residue. Said residue is either a native purinyl or pyrimidyl including adeninyl, uracilyl, thyminyl, cytosinyl and methylcytosinyl, preferably adeninyl or uracilyl. Or it is a derivative of said native purinyl or pyrimidyl residues. Said derivatives include the products of ring hydration, especially 2,3-dihydro-uracilyl; oxa-analogons of the native purinyls or pyrimidinyls containing at least one nitrogen atom in the ring (namely the nitrogen connecting the ring to the ribose unit; and substituted purinyls or pyrimidinyls, oxa-analogons or hydration products, wherein
   (i) the ring hydrogens and/or -NH₂ groups are substituted with a halogen, a C₁-C₃-alkoxyl, C₁-C₃-alkyl, C₁-C₃-alkenyl, or C₁-C₃-alkinyl group;
   (ii) the oxygen atoms in the pyrimidinyl ring carbonyl groups are replaced by -S-R4, =NH, or -N(R3)₂ or by a double bond with the adjacent atom; and/or
   (iii) the hydrogens of the -NH₂-groups in purinyls or cytosinyls are replaced by one or more C₁-C₃-alkyl.
B preferably represents uracilyl or adeninyl or a derivative thereof. Of said derivatives, 2,3-dihydrouracilyl, which resembles uracilyl very closely, is preferred. Even more preferred are native purinyl or pyrimidinyl residues, especially native uracilyl and adeninyl. The most preferred residue B is native uracilyl.
B is connected with the ribose moiety via one of the ring nitrogen atoms, preferably via the N-1 of the pyrimidinyl residues or the N-9 of the purinyl residues. More preferably, B is 1-uracilyl or 9-adeninyl, more preferably is 1-uracilyl.

R1 represent independently from each other residues selected from the group consisting of hydroxyl, hydrogen, C₁-C₃-alkoxyl, C₁-C₃-alkyl, C₁-C₃-alkenyl, C₁-C₃-alkinyl, C₁-C₃-acyl, halogen, or commonly form a double bond with one of the vicinal C atoms or an acetyl or ketal ring with each other. Preferably, at least one R1 is OH and the other R1 is H or OH. More preferred, both R1 are OH.
R2 is -(CH₂)₀₋₂- or phenyliden. If R2 is phenyliden, it may be connected in o-, m- or p-position with the other elements of the compound according to present invention. However, the p-connection is preferred.

R5 is a carbonyl or methylidene (-CH₂-) group. It is preferably a carbonyl group, thus forming an amide bound with the adjacent amine function.

The ring atoms of the ribose unit are chiral. The spatial orientation of their substituents is arbitrary. However, an orientation like in the native ribose furanoside of nucleotides is preferred. Said orientation is the one represented in the following formula of a preferred compound of present invention: 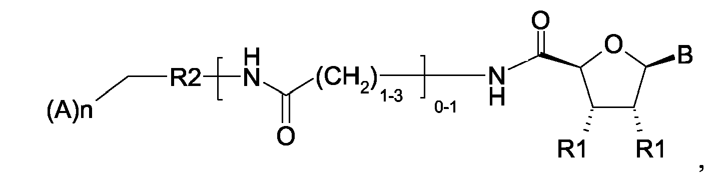 wherein all variables are defined as above.

A represents a -PO(OR3)₂, -SO2(OR3), or -(CH₂)ₘ-COOR4 residue, wherein m is an integer from 0 to 2, R3 is a C₁-C₃-alkyl and R4 is selected from the group consisting of hydrogen and C₁-C₃-alkyl.

In one preferred aspect of the invention, A represents a -PO(OR3)₂ residue. If n is 1, this means that there is one terminal -PO(OR3)₂ group in the compound of present invention. If n is 2, there are two of them. However, it is preferred that n is 1. Furthermore in said preferred aspect, R3 is preferably an ethyl or methyl moiety, most preferably an ethyl moiety.

Thus, an especially preferred compound of present invention is represented by the following formula: 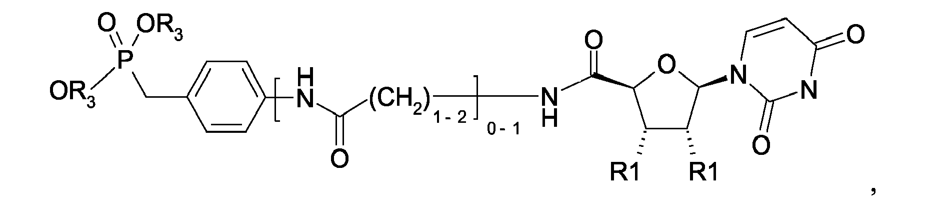 wherein preferably
(i) at least one R1 is OH and the other R1 is H or OH, more preferably both R1 are OH; and/or
(ii) R3 is ethyl.

As far as residue A is concerned, in a further preferred aspect of present invention said residue A represents a -(CH₂)ₘ-COOR4 residue. In this aspect, moreover, R4 is H and/or n is 2. Even more preferred, n is 2 and m is 0 in one of the two -(CH₂)ₘ-COOR4 groups.

The following compounds of embodiment (1) are especially preferred:

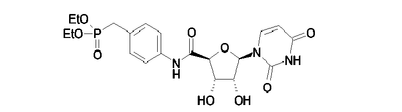

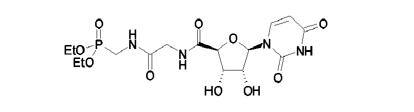

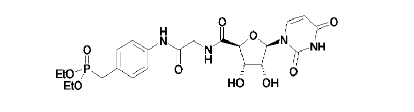

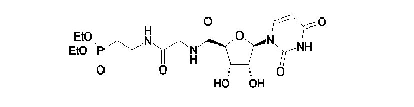

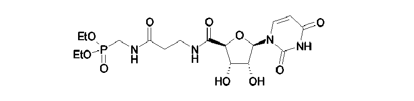

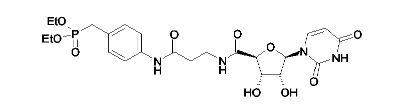

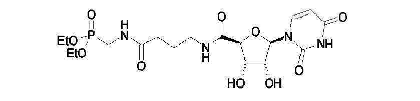

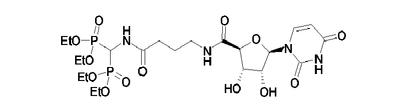

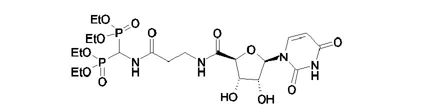

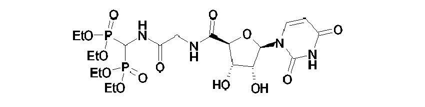

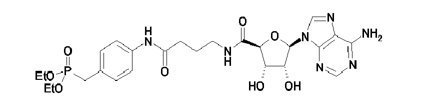

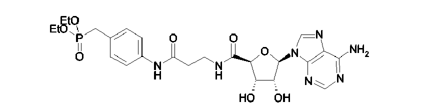

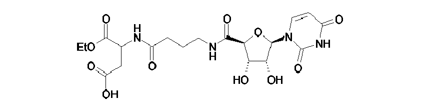

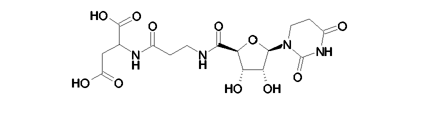

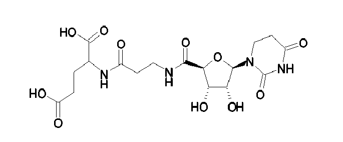

Of these, the compound which is represented by the formula 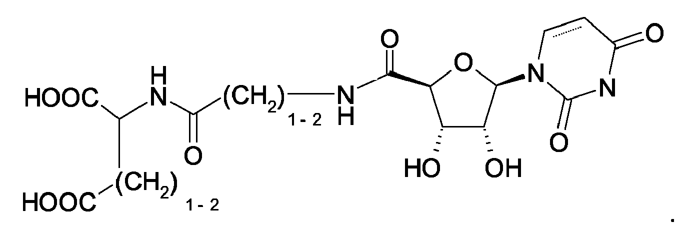 and the compound which is represented by the formula 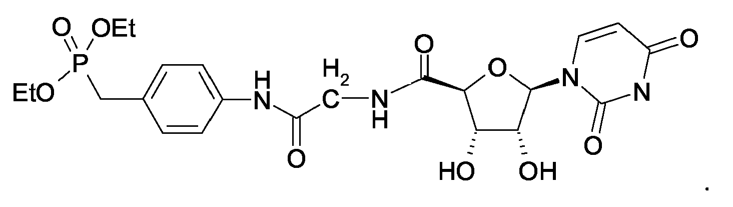 are the most preferred ones. The latter one is an excellent inhibitor of NTPDase (compare Tab. 1) and is therefore even more preferred.

The compounds of present invention are probably competitive inhibitors of NTPDases. Thus, they are of interest for any therapy wherein an activation of P2 receptors is advantageous.

The pharmaceutical composition of embodiment (2) is preferably the medicament of embodiment (3). Furthermore, said medicament of embodiment (3) is preferably for therapy of dry eye disease, respiratory diseases, cystic fibrosis, inflammatory diseases, diseases of the immune system, gastrointestinal diseases, kidney disorders, cancer, and brain diseases. Especially preferred is a medicament for therapy of cancer.

The pharmaceutical composition and the medicament of present invention are applicable in any way allowing the incorporation of the compounds of present invention. As the compounds of present invention are more stable to hydrolysis than compounds containing a phosphate chain, their oral application is preferred.

A further preferred aspect of present invention is the use of the compounds of embodiment (1) in the method of embodiment (5). Especially preferred is the use in a luciferase assay. The known NTPDase inhibitor ARL 67156 is metabolically unstable towards ecto-nucleotide pyrophosphatases (E-NPP). It can be applied as a pharmacological tool but is not suitable in assays where the luciferase assay is used for the quantification of ATP concentrations since it interferes with that assay. It was shown that the compounds of embodiment (1) do not interfere with the luciferase assay for ATP determination. They have therefore major advantages as pharmacological tools in comparison to ARL 67156 and other known NTPDase inhibitors.

The method (6) preferably comprises the following steps: reacting a compound of formula (II) 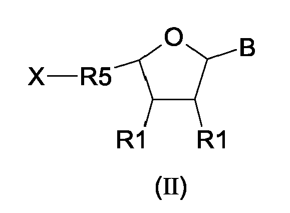 wherein X is a leaving group and all other variables are as defined above, with a compound of formula (III) 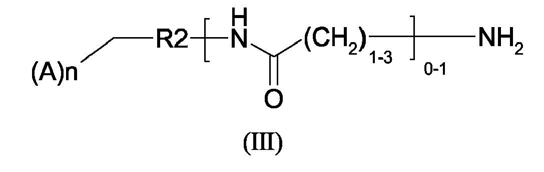 wherein all variables are as defined above. Of course, reactive groups which are not part of said coupling reaction (e.g. the free hydroxy groups of the ribose moiety) are adequately protected beforehand and deprotected after the reaction. Such protection/deprotection reactions are known in the art and exemplified in examples 1 to 20.

The leaving group X is selected from halogen, tosylate, mesylate, and activated esters.

The present invention is described in more detail by reference to the following examples. It should be understood that these examples are for illustrative purpose only and are not to be construed as limiting the invention.

### Examples

All commercially available chemicals and solvents were obtained from various companies (Fluka, Merck, Acros, Sigma-Aldrich). Preparative column chromatography was performed on silica gel 60 (Fluka) 230-400 mesh. Preparative RP-HPLC was performed on a Eurosphere 100 C₁₈ column (250 x 20 mm) with a mixture of MeOH and H₂O at a flow rate of 20 ml/min. ¹H-NMR-, ¹³C-NMR- and ³¹P-NMR-spectra were recorded on a Bruker Avance 500 NMR-spectrometer. Shifts (δ) are given in ppm. The ESI mass spectra were recorded on an API 2000 (Applied Biosystems, Darmstadt, Germany) mass spectrometer at the Pharmaceutical Institute Poppelsdorf, University of Bonn, Germany (ESI, sprayed from a 10⁻⁵ M solution in 2 mM NH₄OAc/Me 0.75:0.25, flow rate 10 µl/min).

### Example 1: Uridine-5'-carboxylic acid[p-(diethylphosphonomethyl)phenyl]amide

Under an atmosphere of argon, 2' ,3' -anisylideneuridine- 5' -carboxylic acid (1 mmol, 376 mg), PyBOP^{®} (1.1 mmol, 572 mg) and 1-hydroxybenzotriazole (1.1 mmol, 149 mg) are dissolved in 2 ml of dry DMF at 0 °C. Diisopropylethylamine (1.1 mmol, 143 mg) and, after one minute, aminobenzylphosphonic acid diethyl ester (2 mmol, 1215 mg), dissolved in 2 ml of dry DMF, are added sequentially via a syringe to the solution. Vigorous stirring is continued for 24 hours at ambient temperature. The volatiles are removed in vacuo at 40 °C and the residue purified by silica gel column chromatography using dichloromethane : methanol (40 : 1) as an eluent. The product is isolated by rotary evaporation at 40 °C and recrystallized from diethyl ether.

Deprotection of the ribose moiety is performed by stirring 2',3'-anisylideneuridine-5'-amide (100 mg) in a mixture of dichloromethane (3 ml), trifluoroacetic acid (0.15 ml) and water (one drop) at ambient temperature. After two hours, the crude product is precipitated by the addition of diethyl ether (50 ml), filtered off, dissolved in 7 ml of water : methanol (75 : 25) and purified by RP-HPLC using a gradient of water : methanol of 75 : 25 to water : methanol 0 : 100. Uridine-5'-carboxylic acid [p-(diethylphosphonomethyl)phenyl]amide is obtained by lyophilization.

Yield over two steps: 230 mg, 48 %, white amorphous powder.
¹H-NMR (500 MHz, MeOD), δ (ppm) 8.17 (d, 1H, ³J = 7.90 Hz, H-6), 7.67 (d, 2H, ³J = 8.85 Hz, 2 x CH (aromate, p-aminobenzylphosphonate)), 7.32 (dd, 2H, ³J = 8.80 Hz and ⁴J = 2.80 Hz, 2 x CH (aromate, p-aminobenzylphosphonate)), 5.85 (d, 1H, ³J = 6.30 Hz, H-1'), 5.80 (d, 1H, ³J = 8.20 Hz, H-5), 4.61 (pseudo-q, 1H, ³J = 5.05 Hz and ³J = 5.95 Hz, H-2'), 4.56 (d, 1H, ³J = 3.20 Hz, H-4'), 4.35 (pseudo-q, 1H, ³J = 3.20 Hz and ³J = 5.05 Hz, H-3'), 4.10 - 4.04 (2 x q, 4H, 2 x OCH₂), 3.26 (d, 2H, ²J_{H,P} = 21.45 Hz, CH₂P (p-aminobenzylphosphonate)), 1.30 (t, 6H, 2 x CH₃).
¹³C-NMR (500 MHz, MeOD), δ (ppm) 170.8 (C=O), 166.5 (C-4), 153.1 (C-2), 145.2 (C-2), 138.5 (C-NH (aromate, p-aminobenzylphosphonate, para-position)), 131.7 (2 x CH (aromate, p-aminobenzylphosphonate, ortho-position)), 129.2 (d, ²J_{C,P} = 38.7 Hz, C-CH₂P (aromate, p-aminobenzylphosphonate)), 121.7 (2 x CH (aromate, p-aminobenzylphosphonate, meta-position)), 103.5 (C-5), 94.1 (C-1'), 86.1 (C-4), 75.1 (C-2), 73.8 (C-3), 64.1 (2 x OCH₂), 33.5 (d, ¹J_{C,P} = 551.2 Hz, CH₂-P (p-aminobenzylphosphonate)), 17.0 (2 x CH₃).
³¹P-NMR (500 MHz, MeOD), δ (ppm) 26.7 (s).
MS (ESI):
m/z +1: 484.1
m/z -1: 482.3

### Example 2: Uridine-5'-carboxylic acid[p-(diethylphosphonomethyl)phenylcarbamoylmethyl]amide

In a dry vessel, N-tert-butyloxycarbonylglycine (10 mmol, 1750 mg) is dissolved in 10 ml of dry THF and cooled to -25 °C. Subsequently, N-methylmorpholine (10 mmol, 1010 mg) and isobutyl chloroformiate (10 mmol, 1360 mg) are sequentially added under vigorous stirring. Immediately after the formation of a white precipitate (N-methylmorpholine hydrochloride) a solution of aminobenzylphosphonic acid diethyl ester (11 mmol, 2673 mg) in dry THF (10 ml) is added. The resulting mixture is allowed to warm to ambient temperature. After three hours, the volatiles are removed by rotary evaporation at 40 °C, the residue is dissolved in 10 ml of water and adjusted to pH 1 (10 % aq. NaHSO₄ solution) and extracted with ethyl acetate (3 x 50 ml). The combined organic layers are washed with saturated aq. Na₂CO₃ solution (3 x 20 ml) and subsequently with water (3 x 20 ml), dried over Na₂SO₄, and evaporated to dryness.

The residue (boc-protected amide) is dissolved in 8 ml of dry 4N HCl-dioxane solution and stirred for two hours at ambient temperature. p-(Aminomethylcarboxamido)benzylphosphonic acid diethyl ester hydrochloride is precipitated by addition of 50 ml of diethyl ether, filtered off and thoroughly washed with diethyl ether.

Yield over two steps: 3100 mg, 92 %, white crystals.

Under an atmosphere of argon, 2' ,3' -anisylideneuridine-5'-carboxylic acid (1 mmol, 376 mg), HCTU^{®} (1.1 mmol, 455 mg) and 1-hydroxybenzotriazole (1.1 mmol, 149 mg) are dissolved in 2 ml of dry DMF at ambient temperature. Diisopropylethylamine (1.1 mmol, 143 mg) and, after one minute, p-(aminomethylcarboxamido)benzylphosphonic acid diethyl ester hydrochloride (2 mmol, 672 mg), dissolved in a mixture of dry DMF (2 ml) and diisopropylethylamine (0.5 ml), are added sequentially via a syringe to the solution. Vigorous stirring is continued for 24 hours at ambient temperature. The volatiles are removed in vacuo at 40 °C and the residue is purified by silica gel column chromatography using dichloromethane : methanol (40 : 1). The product is isolated by rotary evaporation at 40 °C and recrystallized from diethyl ether.

Deprotection of the ribose moiety is performed by stirring 2',3'-anisylideneuridine-5'-amide (100 mg) in a mixture of dichloromethane (3 ml), trifluoroacetic acid (0.15 ml) and water (one drop) at ambient temperature. After two hours, the crude product is precipitated by the addition of diethyl ether (50 ml), filtered off, dissolved in 7 ml of water : methanol (75 : 25) and purified by RP-HPLC using a gradient of water : methanol from 75 : 25 to water : methanol 0 : 100. Uridine-5'-carboxylic acid [p-(diethylphosphonomethyl)phenylcarbamoylmethyl]amide is isolated by lyophilization.

Yield over two steps: 310 mg (57 %), white amorphous powder
¹H-NMR (500 MHz, MeOD), δ 8.18 (d, 1H, ³J = 7.90 Hz, H-6), 7.58 (d, 2H, ³J = 8.85 Hz, 2 x CH (aromate, p-aminobenzylphosphonate)), 7.31 (dd, 2H, ³J = 8.80 Hz and ⁴J = 2.80 Hz, 2 x CH (aromate, p-aminobenzylphosphonate)), 6.02 (d, 1H, ³J = 6.30 Hz, H-1'), 5.78 (d, 1H, ³J = 8.20 Hz, H-5), 4.51 (d, 1H, ³J = 3.20 Hz, H-4'), 4.47 (pseudo-q, 1H, ³J = 5.05 Hz und ³J = 5.95 Hz, H-2'), 4.41 (pseudo-q, 1H, ³J = 3.20 Hz and ³J = 5.05 Hz, H-3'), 4.19 - 4.01 (AB-system with A d and B d, partly covered by 2 x O-CH₂, 2H, ²J_{A},_{B} = 16.35 Hz, N-CH₂ (carbamoylmethylamine)), 4.09 - 4.01 (2 x q, 4H, 2 x O-CH₂), 3.25 (d, 2H, ²J_{H,P} = 21.45 Hz, CH₂-P (p-aminobenzylphosphonate)), 1.29 (t, 6H, 2 x CH₃).
¹³C-NMR (500 MHz, MeOD), δ 173.2 (C=O), 169.5 (C=O), 166.4 (C-4), 153.1 (C-2), 144.3 (C-6), 138.9 (C-NH (p-aminobenzylphosphonate, para-position)), 131.7 (2 x CH (p-aminobenzylphosphonate, ortho-position)), 128.7 (d, ²J_{C,P} = 37.7 Hz, C-CH₂P (p-aminobenzylphosphonate)), 121.5 (2 x CH (p-aminobenzylphosphonate, meta-position)), 103.5 (C-5), 92.2 (C-1'), 85.3 (C-4), 74.9 (C-2), 74.1 (C-3), 64.1 und 64.0 (2 x O-CH₂), 43.9 (N-CH₂ (carbamoylmethylamine)), 33.4 (d, ¹J_{C,P} = 550.2 Hz, CH₂-P (p-aminobenzylphosphonate)), 17.0 und 16.9 (2 x CH₃).
³¹P-NMR (500 MHz, MeOD), δ 26.7 (s).
MS (ESI)
m/z +1: 541.0
m/z-1: 539.3

### Example 3: Uridine-5'-carboxylic acid [2-(p-(diethylphosphonomethyl)phenylcarbamoyl)ethyl]amide

In a dry vessel, N-tert-butyloxycarbonyl-β-alanine (10 mmol, 1890 mg) is dissolved in 10 ml of dry THF and cooled to -25 °C. Subsequently, N-methylmorpholine (10 mmol, 1010 mg) and isobutyl chloroformiate (10 mmol, 1360 mg) are sequentially added under vigorous stirring. Immediately after the formation of a white precipitate (N-methylmorpholine hydrochloride) a solution of aminobenzylphosphonic acid diethyl ester (11 mmol, 2673 mg) in dry THF (10 ml) is added. The resulting mixture is allowed to warm to ambient temperature. After three hours, the volatiles are removed by rotary evaporation at 40 °C, the residue is dissolved in 10 ml of water and adjusted to pH 1 (10% aq. NaHSO₄ solution) and extracted with ethyl acetate (3 x 50 ml). The combined organic layers are washed with satured aq. Na₂CO₃ solution (3 x 20 ml) and subsequently with water (3 x 20 ml), dried over Na₂SO₄, and evaporated to dryness.

The residue (boc-protected amide) is dissolved in 8 ml of dry 4N HCl-dioxane solution and stirred for two hours at ambient temperature. p-(Aminoethylcarboxamido)benzylphosphonic acid diethyl ester hydrochloride is precipitated by addition of 50 ml of diethyl ether, filtered off and thoroughly washed with diethyl ether.

Yield over two steps: 3000 mg, 86 %, white crystals.

Under an atmosphere of argon, 2' ,3' -anisylideneuridine-5 -carboxylic acid (1 mmol, 376 mg), HBTU^{®} (1.1 mmol, 428 mg) and 1-hydroxybenzotriazole (1.1 mmol, 149 mg) are dissolved in 2 ml of dry DMF at ambient temperature. Diisopropylethylamine (1.1 mmol, 143 mg) and, after one minute, p-(aminoethylcarboxamido)benzylphosphonic acid diethyl ester hydrochloride (2 mmol, 700 mg), dissolved in a mixture of dry DMF (2 ml) and diisopropylethylamine (0.5 ml), are added sequentially via a syringe to the solution. Vigorous stirring is continued for 24 hours at ambient temperature. The volatiles are removed in vacuo at 40 °C and the residue is purified by silica gel column chromatography using dichloromethane/methanol (40:1). The product is isolated by rotary evaporation at 40 °C and recrystallized from diethyl ether. Deprotection of the ribose moiety is performed by stirring 2',3'-anisyiideneuridine-5'-amide (100 mg) in a mixture of dichloromethane (3 ml), trifluoroacetic acid (0.15 ml) and water (one drop) at ambient temperature. After two hours, the crude product is precipitated by addition of diethyl ether (50 ml), filtered off, dissolved in 7 ml of water/methanol (75 : 25) and purified by RP-HPLC using a gradient of water : methanol from 75 : 25 to water : methanol 0:100. Uridine-5'-carboxylic acid [2-(p-(diethylphosphonomethyl)phenylcarbamoyl)ethyl]amide is isolated by lyophilisation.

Yield over two steps: 350 mg (63 %), white amorphous powder
¹H-NMR (500 MHz, MeOD), δ 8.08 (d, 1H, ³J = 8.20 Hz, H-6), 7.55 (d, 2H, ³J = 7.90 Hz, 2 x CH (aromate, p-aminobenzylphosphonate)), 7.29 (dd, 2H, ³J = 8.50 Hz and ⁴J = 2.85 Hz, 2 x CH (aromate, p-aminobenzylphosphonate)), 5.92 (d, 1H, ³J = 6.30 Hz, H-1'), 5.72 (d, 1H, ³J = 7.90 Hz, H-5), 4.41 (pseudo-q, 1H , ³j = 5.05 Hz and ³J = 6.30 Hz, H-2'), 4.40 (d, 1H, ³J = 2.85, H-4'), 4.28 (pseudo-q, 1H, ³J = 5.05 Hz and ²J = 2.85 Hz, H-3'), 4.10 - 4.03 (2 x q, 4H, 2 x O-CH₂), 3.69 - 3.56 (AA'XX'-system, 2H, ³J_{A,X} = 6.30 Hz and ³J_{A,X'} = 6.95 Hz and ³J_{A',X} = 6.95 Hz and ³J_{A',X'} = 6.60 Hz and ²J_{A,A'} = 16.10 Hz, N-CH₂ (carbamoylethylamine)), 3.23 (d, 2H, ²J_{H,P} = 21.45 Hz, CH₂P (p-aminobenzylphosphonate)), 2.65 (AA'XX'-system, 2H, ³J_{X,A} = 6.30 Hz and ³J_{X,A'} = 6.60 Hz and ³J_{X',A} = 6.65 Hz and ³J_{X',A'} = 6.60 Hz and ²J_{X',X'} = 15.75 Hz, O=C-CH₂ (carbamoylethylamine)), 1.29 (t, 6H, 2 x CH₃).
¹³C-NMR (500 MHz, MeOD), δ 172.6 (C=O), 172.2 (C=O), 166.3 (C-4), 152.9 (C-2), 144.3 (C-6), 139.1 (C-NH (aromate, p-aminobenzylphosphonate, para-position)), 131.7 (2 x CH p-aminobenzylphosphonate, ortho-position)), 128.6 (d, ²J_{C,P} = 37.7 Hz, C-CH₂P (p-aminobenzylphosphonate)), 121.5 (2 x CH (p-aminobenzylphosphonate, meta-position)), 103.4 (C-5), 92.4 (C-1'), 85.4 (C-4), 74.9 (C-2), 74.1 (C-3), 64.1 and 64.0 (2 x O-CH₂), 37.6 (N-CH₂ (carbamoylethylamine)), 37.1 (O=C-CH₂ (carbamoylethylamine)), 33.4 (d, ¹J_{C,P} = 550.2 Hz, CH₂-P (p-aminobenzylphosphonate)), 16.9 and 16.8 (2 x CH₃).
³¹P-NMR (500 MHz, MeOD), δ 26.8 (s).
MS (ESI):
m/z +1: 555.3
m/z -1: 553.3

### Example 4: Uridine-5'-carboxylic acid [3-(p-(diethylphosphonomethyl)phenylcarbamoyl)propyl]amide

In a dry vessel, N-tert-butyloxycarbonyl-y-aminobutyric acid (10 mmol, 2030 mg) is dissolved in 10 ml of dry THF and cooled to -25 °C. Subsequently, N-methylmorpholine (10 mmol, 1010 mg) and isobutyl chloroformiate (10 mmol, 1360 mg) are sequentially added under vigorous stirring. Immediately after the formation of a white precipitate (N-methylmorpholine hydrochloride) a solution of aminobenzylphosphonic acid diethyl ester (11 mmol, 2673 mg) in dry THF (10 ml) is added. The resulting mixture is allowed to warm to ambient temperature. After three hours, the volatiles are removed by rotary evaporation at 40 °C, the residue is dissolved in 10 ml of water and adjusted to pH 1 (10% aq. NaHSO₄ solution) and extracted with ethyl acetate (3 x 50 ml). The combined organic layers are washed with satured aq. Na₂CO₃ solution (3 x 20 ml) and subsequently with water (3 x 20 ml), dried over Na₂SO₄, and evaporated to dryness.

The residue (boc-protected amide) is dissolved in 8 ml of dry 4N HCl-dioxane solution and stirred for two hours at ambient temperature. p-(Aminopropylcarboxamido)benzylphosphonic acid diethyl ester hydrochloride is precipitated by addition of 50 ml of diethyl ether, filtered off and thoroughly washed with diethyl ether.

Yield over two steps: 3300 mg, 91 %, white crystals.

Under an atmosphere of argon, 2' ,3' -anisylideneuridine-5'-carboxylic acid (1 mmol, 376 mg), HBTU^{®} (1.1 mmol, 482 mg) and 1-hydroxybenzotriazole (1.1 mmol, 149 mg) are dissolved in 2 ml of dry DMF at ambient temperature. Diisopropylethylamine (1.1 mmol, 143 mg) and, after one minute, p-(aminopropylcarboxamido)benzylphosphonic acid diethylester hydrochloride (2 mmol, 728 mg), dissolved in a mixture of dry DMF (2 ml) and diisopropylethylamine (0.5 ml), are added sequentially via a syringe to the solution. Vigorous stirring is continued for 24 hours at ambient temperature. The volatiles are removed in vacuo at 40 °C and the residue is purified by silica gel column chromatography using dichloromethane/methanol (40:1). The product is isolated by rotary evaporation at 40 °C and recrystallized from diethyl ether. Deprotection of the ribose moiety is performed by stirring 2',3'-anisylideneuridine-5'-amide (100 mg) in a mixture of dichloromethane (3 ml), trifluoroacetic acid (0.15 ml) and water (one drop) at ambient temperature. After two hours, the crude product is precipitated by addition of diethyl ether (50 ml), filtered off, dissolved in 7 ml of water/methanol (75 : 25) and purified by RP-HPLC using a gradient of water : methanol from 75 : 25 to water : methanol 0 : 100. Uridine-5'-carboxylic acid [3-(p-(diethylphosphonomethyl)phenylcarbamoyl)propyl]amide is isolated by lyophilisation.

Yield over two steps: 340 mg (60 %), white amorphous powder
¹H-NMR (500 MHz, MeOD), δ 8.11 (d, 1H, ³J = 8.20 Hz, H-6), 7.55 (d, 2H, ³J = 7.90 Hz, 2 x CH (aromate, p-aminobenzylphosphonate)), 7.28 (dd, 2H, ³J = 8.50 Hz and ⁴J = 2.85 Hz, 2 x CH (aromate, p-aminobenzylphosphonate)), 5.86 (d, 1H, ³J = 6.30 Hz, H-1'), 5.75 (d, 1H, ³J = 7.90 Hz, H-5), 4.45 (pseudo-q, 1H, ³J = 5.05 Hz and ³J = 6.30 Hz, H-2'), 4.39 (d, 1H, ³J = 2.85, H-4'), 4.27 (pseudo-q, 1H, ³J = 5.05 Hz and ²J = 2.85 Hz, H-3'), 4.09 - 4.03 (2 x q, 4H, 2 x O-CH₂), 3.38 (t, partly under solvent peak, 2H, ³J = 7.25 Hz, N-CH₂ (carbamoylpropylamine)), 3.23 (d, 2H, ²J_{H,P} = 21.45 Hz, CH₂-P (p-aminobenzylphosphonate)), 2.45 (t, 2H, ³J = 7.25 Hz, O=C-CH₂ (carbamoylpropylamine)), 1.95 (tt, 2H, ³J = 7.25 Hz, CH₂ (carbamoylpropylamine)), 1.29 (t, 6H, 2 x CH₃).
¹³C-NMR (500 MHz, MeOD), δ 174.0 (C=O), 172.7 (C=O), 166.3 (C-4), 152.9 (C-2), 144.6 (C-6), 139.2 (C-NH (p-aminobenzylphosphonate, para-position)), 131.6 (2 x CH (p-aminobenzylphosphonate, ortho-position)), 128.5 (d, ²J_{C,P} = 37.7 Hz, C-CH₂P (p-aminobenzylphosphonate)), 121.4 (2 x CH (p-aminobenzylphosphonate, meta-position)), 103.3 (C-5), 93.1 (C-1'), 85.4 (C-4), 74.8 (C-2), 74.1 (C-3), 64.0 (2 x O-CH₂), 40.1 (N-CH₂ (carbamoylpropylamine)), 35.5 (O=C-CH₂ (carbamoylpropylamine)), 33.4 (d, ¹J_{C,P} = 550.2 Hz, CH₂-P (p-aminobenzylphosphonate)), 26.6 (CH₂ (carbamoylpropylamine)), 16.9 and 16.8 (2 x CH₃).
³¹P-NMR (500 MHz, MeOD), δ 26.8 (s).
MS (ESI):
m/z +1: 569.2
m/z -1: 567.3

### Example 5: Uridine-5'-carboxylic acid (diethylphosphonomethylcarbamoymethyl)amide

In a dry vessel, N-tert-butyloxycarbonylglycine (10 mmol, 1750 mg) is dissolved in 10 ml of dry THF and cooled to -25 °C. Subsequently, N-methylmorpholine (10 mmol, 1010 mg) and isobutyl chloroformiate (10 mmol, 1360 mg) are sequentially added under vigorous stirring. Immediately after the formation of a white precipitate (N-methylmorpholine hydrochloride) a solution of aminomethylphosphonic acid diethylester oxalate (11 mmol, 2827 mg) in THF (10 ml) and 1N-NaOH (11 ml), pre-cooled on ice, is added. The resulting mixture is allowed to warm to ambient temperature. After three hours, the volatiles are removed by rotary evaporation at 40 °C, the residue is dissolved in 10 ml of water and adjusted to pH 1 (10% aq. NaHSO₄ solution) and extracted with ethyl acetate (3 x 50 ml). The combined organic layers are washed with satured aq. Na₂CO₃ solution (3 x 20 ml) and subsequently with water (3 x 20 ml), dried over Na₂SO₄, and evaporated to dryness.

The residue (boc-protected amide) is dissolved in 8 ml of dry 4N HCl-dioxane solution and stirred for two hours at ambient temperature. Aminomethylcarboxamidomethylphosphonic acid diethyl ester hydrochloride is precipitated by addition of 50 ml of diethyl ether, filtered off and thoroughly washed with diethyl ether.

Yield over two steps: 2200 mg, 85 %, white crystals.

Under an atmosphere of argon, 2',3'-anisylideneuridine-5'-carboxylic acid (1 mmol, 376 mg), HCTU^{®} (1.1 mmol, 455 mg) and 1-hydroxybenzotriazole (1.1 mmol, 149 mg) are dissolved in 2 ml of dry DMF at ambient temperature. Diisopropylethylamine (1.1 mmol, 143 mg) and, after one minute, aminomethylcarboxamidomethylphosphonic acid diethyl ester hydrochloride (2 mmol, 522 mg), dissolved in a mixture of dry DMF (2 ml) and diisopropylethylamine (0.5 ml), are added sequentially via a syringe to the solution. Vigorous stirring is continued for 24 hours at ambient temperature. The volatiles are removed in vacuo at 40 °C and the residue is purified by silica gel column chromatography using dichloromethane/methanol (40:1). The product is isolated by rotary evaporation at 40 °C and recrystallized from diethyl ether.

Deprotection of the ribose moiety is performed by stirring 2',3'-anisylideneuridine-5'-amide (100 mg) in a mixture of dichloromethane (3 ml), trifluoroacetic acid (0.15 ml) and water (one drop) at ambient temperature. After two hours, the crude product is precipitated by addition of diethyl ether (50 ml), filtered off, dissolved in 7 ml of water/methanol (75 : 25) and purified by RP-HPLC using a gradient of water : methanol from 75 : 25 to water : methanol 0 : 100. Uridine-5' -carboxylic acid (diethylphosphonomethylcarbamoylmethyl)amide is isolated by lyophilisation.

Yield over two steps: 190 mg (41 %), white amorphous powder
¹H-NMR (500 MHz, MeOD), δ 8.10 (d, 1H, ³J = 7.85 Hz, H-6), 5.91 (d, 1H, ³J = 5.95 Hz, H-1'), 5.77 (d, 1H, ³J = 7.85 Hz, H-5), 4.50 (pseudo-q, 1H, ³J = 5.35 Hz and ³J = 5.70 Hz, H-2'), 4.48 (d, 1H, ³J = 2.85 Hz, H-4'), 4.44 (pseudo-q, 1H, ³J = 5.0 Hz and ³J = 3.15 Hz, H-3 '), 4.22 - 4.15 (2 x q, 4H, 2 x O-CH₂), 4.10 - 3.83 (AB-system with A d and B d, 2H, ²J = 17.00 Hz, N-CH₂ (carbamoylmethylamine)), 3.84 - 3.72 (AB-system with A dd and B dd, 2H, ²J_{H,P} = 11.65 Hz and ²J_{A},_{B} = 15.75 Hz, N-CH₂ (aminomethylphosphonate)), 1.35 (2 x t, 6H, 2 x CH₃).
¹³C-NMR (500 MHz, MeOD), δ 173.2 (C=O), 171.6 (C=O), 166.4 (C-4), 153.1 (C-2), 144.9 (C-6), 103.5 (C-5), 93.9 (C-1'), 85.7 (H-4'), 74.9 (H-2'), 74.2 (H-3'), 64.5 (2 x OCH₂), 43.4 (N-CH₂ (carbamoylmethylamine)), 35.7 (d, ¹J_{C,P} = 630.5 Hz, CH₂-P (aminomethylphosphonate)), 17.0 (2 x CH₃).
³¹P-NMR (500 MHz, MeOD), δ 22.1 (s).
MS (ESI):
m/z +1: 465.1
m/z -1: 463.1

### Example 6: Uridine-5'-carboxylic acid [2-(diethylphosphonomethylcarbamoyl)-ethyl]amide

In a dry vessel, N-tert-butyloxycarbonyl-β-alanine (10 mmol, 1750 mg) is dissolved in 10 ml of dry THF and cooled to -25 °C. Subsequently, N-methylmorpholine (10 mmol, 1010 mg) and isobutyl chloroformiate (10 mmol, 1360 mg) are sequentially added under vigorous stirring. Immediately after the formation of a white precipitate (N-methylmorpholine hydrochloride) a solution of aminomethylphosphonic acid diethyl ester oxalate (11 mmol, 2827 mg) in THF (10 ml) and 1N-NaOH (11 ml), pre-cooled on ice, is added. The resulting mixture is allowed to warm to ambient temperature. After three hours, the volatiles are removed by rotary evaporation at 40 °C, the residue is dissolved in 10 ml of water and adjusted to pH 1 (10% aq. NaHSO₄ solution and extracted with ethyl acetate (3 x 50 ml). The combined organic layers are washed with satured aq. Na₂CO₃ solution (3 x 20 ml) and subsequently with water (3 x 20 ml), dried over Na₂SO₄, and evaporated to dryness.

The residue (boc-protected amide) is dissolved in 8 ml of dry 4N HCl-dioxane solution and stirred for two hours at ambient temperature. 2-Aminoethylcarboxamidomethylphosphonic acid diethyl ester hydrochloride is precipitated by addition of 50 ml of diethyl ether, filtered off and thoroughly washed with diethyl ether.
Yield over two steps: 2000 mg, 73 %, clay.

Under an atmosphere of argon, 2',3'-anisylideneuridine-5'-carboxylic acid (1 mmol, 376 mg), PyBOP^{®} (1.1 mmol, 455 mg) and 1-hydroxybenzotriazole (1.1 mmol, 149 mg) are dissolved in 2 ml of dry DMF at ambient temperature. Diisopropylethylamine (1.1 mmol, 143 mg) and, after one minute, 2-aminoethylcarboxamidomethylphosphonic acid diethyl ester hydrochloride (2 mmol, 578 mg), dissolved in a mixture of dry DMF (2 ml) and diisopropylethylamine (0.5 ml), are added sequentially via a syringe to the solution. Vigorous stirring is continued for 24 hours at ambient temperature. The volatiles are removed in vacuo at 40 °C and the residue is purified by silica gel column chromatography using dichloromethane/methanol (40:1). The product is isolated by rotary evaporation at 40 °C and recrystallized from diethyl ether.

Deprotection of the ribose moiety is performed by stirring 2',3'-anisylideneuridine-5'-amide (100 mg) in a mixture of dichloromethane (3 ml), trifluoroacetic acid (0.15 ml) and water (one drop) at ambient temperature. After two hours, the crude product is precipitated by addition of diethyl ether (50 ml), filtered off, dissolved in 7 ml of water : methanol (75 : 25) and purified by RP-HPLC using a gradient of water : methanol from 75 : 25 to water : methanol 0 : 100. Uridine-5'-carboxylic acid [2-(diethylphosphonomethylcarbamoyl)ethyl]-amide is isolated by lyophilisation.

Yield over two steps: 170 mg (36 %), white amorphous powder
¹H-NMR (500 MHz, MeOD), δ 8.13 (d, 1H, ³J = 8.20 Hz, H-6), 5.91 (d, 1H, ³J = 6.00 Hz, H-1'), 5.79 (d, 1H, ³J = 8.20 Hz, H-5), 4.44 (pseudo-q, 1H, ³J = 5.05 Hz and ³J = 5.95 Hz, H-2'), 4.38 (d, 1H, ³J = 3.20 Hz, H-4'), 4.28 (pseudo-q, 1H, ³J = 5.05 Hz and ³J = 3.15 Hz, H-3'), 4.21 - 4.15 (2 x q, 4H, 2 x O-CH₂), 3.74 (d, 2H, ²J_{H,P} = 11.65 Hz, N-CH₂P (aminomethylphosphonate)), 3.51 (AA'XX'-system with A ddd and B ddd, 2H, ³J_{A,X} = 6.65 Hz and ³J_{A',X} = 6.90 Hz and ³J_{A',X} = 6.65 Hz and ³J_{A',X'} = 6.90 Hz and ²J_{A,B} = 17.30 Hz, N-CH₂ (carbamoylethylamine)), 2.53 (AA 'XX' -system not determinable, instead br pseudo-dt, 2H, ³J = 6.60 Hz and ³J = 7.55 Hz, O=C-CH₂ (carbamoylethylamine)), 1.37 (2 x t, 6H, 2 x CH₃).
¹³C-NMR (500 MHz, MeOD), δ 173.7 (C=O), 172.6 (C=O), 166.8 (C-4), 153.2 (C-2), 144.5 (C-6), 103.4 (C-5), 92.8 (C-1'), 85.4 (C-4'), 74.9 (C-2'), 74.0 (C-3'), 64.4 (2 x O-CH₂), 37.1 (N-CH₂ (carbamoylethylamine)), 36.4 (O=C-CH₂ (carbamoylethylamine)), 35.7 (d, ¹J_{C,P} = 582.9 Hz, CH₂-P (aminomethylphosphonate)), 17.0 (2 x CH₃).
³¹P-NMR (500 MHz, MeOD), δ 24.6 (s).
MS (ESI):
m/z + 1: 479.0
m/z -1: 477.1

### Example 7: Uridine-5'-carboxylic acid [3-(diethylphosphonomethylcarbamoyl)-propyl]amide

In a dry vessel, N-tert-butyloxycarbonyl-γ-aminobutyric acid (10 mmol, 2030 mg) is dissolved in 10 ml of dry THF and cooled to -25 °C. Subsequently, N-methylmorpholine (10 mmol, 1010 mg) and isobutyl chloroformiate (10 mmol, 1360 mg) are sequentially added under vigorous stirring. Immediately after the formation of a white precipitate (N-methylmorpholine hydrochloride) a solution of aminomethylphosphonic acid diethyl ester oxalate (11 mmol, 2827 mg) in THF (10 ml) and 1N-NaOH (11 ml), pre-cooled on ice, is added. The resulting mixture is allowed to warm to ambient temperature. After three hours, the volatiles are removed by rotary evaporation at 40 °C, the residue is dissolved in 10 ml of water and adjusted to pH 1 (10% aq. NaHSO₄ solution) and extracted with ethyl acetate (3 x 50 ml). The combined organic layers are washed with satured aq. Na₂CO₃ solution (3 x 20 ml) and subsequently with water (3 x 20 ml), dried over Na₂SO₄, and evaporated to dryness.

The residue (boc-protected amide) is dissolved in 8 ml of dry 4N HCl-dioxane solution and stirred for two hours at ambient temperature. 3-Aminopropylcarboxamidomethylphosphonic acid diethyl ester hydrochloride is precipitated by addition of 50 ml of diethyl ether, filtered off and thoroughly washed with diethyl ether.
Yield over two steps: 2300 mg, 80 %, white crystals.

Under an atmosphere of argon, 2' ,3'-anisylideneuridine-5 -carboxylic acid (1 mmol, 376 mg), PyBOP^{®} (1.1 mmol, 455 mg) and 1-hydroxybenzotriazole (1.1 mmol, 149 mg) are dissolved in 2 ml of dry DMF at ambient temperature. Diisopropylethylamine (1.1 mmol, 143 mg) and, after one minute, 3-aminopropylcarboxamidomethylphosphonic acid diethyl ester hydrochloride (2 mmol, 550 mg), dissolved in a mixture of dry DMF (2 ml) and diisopropylethylamine (0.5 ml), are added sequentially via a syringe to the solution. Vigorous stirring is continued for 24 hours at ambient temperature. The volatiles are removed in vacuo at 40 °C and the residue is purified by silica gel column chromatography using dichloromethane/methanol (40:1). The product is isolated by rotary evaporation at 40 °C and recrystallized from diethyl ether.

Deprotection of the ribose moiety is performed by stirring 2',3'-anisylideneuridine-5'-amide (100 mg) in a mixture of dichloromethane (3 ml), trifluoroacetic acid (0.15 ml) and water (one drop) at ambient temperature. After two hours, the crude product is precipitated by addition of diethyl ether (50 ml), filtered off, dissolved in 7 ml of water : methanol (75 : 25) and purified by RP-HPLC using a gradient of water : methanol from 75 : 25 to water : methanol 0 : 100. Uridine-5'-carboxylic acid [3-(diethylphosphonomethylcarbamoyl)propyl]-amide is isolated by lyophilisation.

Yield over two steps: 310 mg (63%), white amorphous powder
¹H-NMR (500 MHz, MeOD), δ 8.11 (d, 1H, ³J = 7.85 Hz, H-6), 5.84 (d, 1H, ³J = 6.00 Hz, H-1'), 5.78 (d, 1H, ³J = 8.20 Hz, H-5), 4.50 (pseudo-q, 1H, ³J = 5.05 Hz and ³J = 5.95 Hz, H-2'), 4.39 (d, 1H, ³J = 3.15 Hz, H-4'), 4.27 (pseudo-q, 1H, ³J = 5.05 Hz and ³J = 3.15 Hz, H-3'), 4.20 - 4.15 (2 x q, 4H, 2 x O-CH₂), 3.75 (d, 2H, ²J_{H,P} = 11.65 Hz, CH₂-P (aminomethylphosphonate)), 3.38 - 3.26 (AA'XX'-system, partially covered by solvent, 2H, ³J_{A,X} = 6.95 Hz and ³J_{A',X} = 6.60 Hz and ³J_{A,X'} = 6.60 Hz and ²J_{A,A'} = 13.55 Hz, N-CH₂ (carbamoylpropylamine)), 3.32 (pseudo-t, 2H, ³J = 7.25 Hz and ³J = 7.55 Hz, O=C-CH₂ (carbamoylpropylamine)), 1.88 (m, 2H, ³J = 7.25 Hz and ³J = 6.95 Hz, CH₂ (carbamoylpropylamine)), 1.36 (2 x t, 6H, 2 x CH₃).
¹³C-NMR (500 MHz, MeOD), δ 175.5 (C=O), 172.7 (C=O), 166.4 (C-4), 153.0 (C-2), 144.9 (C-6), 103.3 (C-5), 93.6 (C-1'), 85.5 (C-4'), 74.9 (C-2'), 73.9 (C-3), 64.4 (2 x O-CH₂), 39.9 (N-CH₂ (carbamoylpropylamine)), 35.6 (d, ¹J_{C,P} = 627.5 Hz, CH₂-P (aminomethylphosphonate)), 34.3 (O=C-CH₂ (carbamoylpropylamine)), 26.8 (CH₂ (carbamoylpropylamine)), 17.0 (2 x CH₃).
³¹P-NMR (500 MHz, MeOD), δ 24.6 (s).
MS (ESI):
m/z + 1: 493.1
m/z -1: 491.5

### Example 8: Uridine-5'-carboxylic acid (2-diethylphosphonoethylcarbamoylmethyl)amide

In a dry vessel, N-tert-butyloxycarbonylglycine (10 mmol, 1750 mg) is dissolved in 10 ml of dry THF and cooled to -25 °C. Subsequently, N-methylmorpholine (10 mmol, 1010 mg) and isobutyl chloroformiate (10 mmol, 1360 mg) are sequentially added under vigorous stirring. Immediately after the formation of a white precipitate (N-methylmorpholine hydrochloride) a solution of aminoethylphosphonic acid diethylester oxalate (11 mmol, 2981 mg) in THF (10 ml) and 1N-NaOH (11 ml), pre-cooled on ice, is added. The resulting mixture is allowed to warm to ambient temperature. After three hours, the volatiles are removed by rotary evaporation at 40 °C, the residue is dissolved in 10 ml of water and adjusted to pH 1 (10% aq. NaHSO₄ solution) and extracted with ethyl acetate (3 x 50 ml). The combined organic layers are washed with satured aq. Na₂CO₃ solution (3 x 20 ml) and subsequently with water (3 x 20 ml), dried over Na₂SO₄ and evaporated to dryness.

The residue (boc-protected amide) is dissolved in 8 ml of dry 4N HCl-dioxane solution and stirred for two hours at ambient temperature. 2-(Aminomethylcarboxamido)ethylphosphonic acid diethyl ester hydrochloride is precipitated by addition of 50 ml of diethyl ether, filtered off and thoroughly washed with diethyl ether.
Yield over two steps: 1900 mg, 69 %, clay.

Under an atmosphere of argon, 2' ,3' -anisylideneuridine-5' -carboxylic acid (1 mmol, 376 mg), HCTU^{®} (1.1 mmol, 455 mg) and 1-hydroxybenzotriazole (1.1 mmol, 149 mg) are dissolved in 2 ml of dry DMF at ambient temperature. Diisopropylethylamine (1.1 mmol, 143 mg) and, after one minute, 2-(aminomethylcarboxamido)ethylphosphonic acid diethyl ester hydrochloride (2 mmol, 550 mg), dissolved in a mixture of dry DMF (2 ml) and diisopropylethylamine (0.5 ml), are added sequentially via a syringe to the solution. Vigorous stirring is continued for 24 hours at ambient temperature. The volatiles are removed in vacuo at 40 °C and the residue is purified by silica gel column chromatography using dichloromethane/methanol (40:1). The product is isolated by rotary evaporation at 40 °C and recrystallized from diethyl ether.

Deprotection of the ribose moiety is performed by stirring 2',3'-anisylideneuridine-5'-amide (100 mg) in a mixture of dichloromethane (3 ml), trifluoroacetic acid (0.15 ml) and water (one drop) at ambient temperature. After two hours, the crude product is precipitated by addition of diethyl ether (50 ml), filtered off, dissolved in 7 ml of water : methanol (75 : 25) and purified by RP-HPLC using a gradient of water : methanol from 75 : 25 to water : methanol 0 : 100. Uridine-5'-carboxylic acid (2-diethylphosphonoethylcarbamoylmethyl)amide is isolated by lyophilisation.

Yield over two steps: 280 mg (59 %), white amorphous powder
¹H-NMR (500 MHz, MeOD), δ 8.12 (d, 1H, ³J = 8.20 Hz, H-6), 5.93 (d, 1H, ³J = 5.95 Hz, H-1 '), 5.78 (d, 1H, ³J = 8.20 Hz, H-5), 4.48 (pseudo-q, 1H, ³J = 5.35 Hz and ³J = 6.00 Hz, H-2'), 4.48 (d, 1H, ³J = 3.15 Hz, H-4'), 4.40 (pseudo-q, 1H, ³J = 5.05 Hz and ³J = 3.20 Hz, H-3'), 4.19 - 4.12 (2 x q, 4H, 2 x O-CH₂), 4.01 - 3.83 (AB-system with A d and B d, 2H, ²J_{A,B} = 16.70 Hz, N-CH₂ (carbamoylmethylamine)), 3.50 (AA'XX'-system, 2H, ³J_{A,X} = 7.55 Hz and ³J_{A',X} = 5.40 Hz and ³J_{A,X'} = 7.55 Hz and ³J_{A',X'} = 5.40 Hz and ³J_{H,P} = 12.95 Hz and ²J_{A,A'} = 15.15 Hz, N-CH₂ (aminoethylphosphonate)), 2.16 - 2.09 (AA'XX'-system, 2H, ³J_{X,A} = 7.55 and ³J_{X,A'} = 5.35 Hz and ³J_{X',A} = 7.55 and ³J_{X',A'} = 5.35 Hz and ²J_{H,P} = 18.35 Hz and ²J_{X,X'} = 15.15 Hz, CH₂-P (aminoethylphosphonate)), 1.37 (t, 6H, 2 x CH₃).
¹³C-NMR (500 MHz, MeOD), δ 173.2 (C=O), 171.6 (C=O), 166.4 (C-4), 153.0 (C-2), 144.6 (C-6), 103.4 (C-5), 93.1 (C-1'), 85.4 (C-4'), 74.8 (C-2'), 74.1 (C-3'), 63.8 (2 x O-CH₂), 43.5 (N-CH₂ (carbamoylmethylamine)), 34.9 (N-CH₂ (aminoethylphosphonate)), 26.5 (d, ¹J_{C,P} = 553.2 Hz, CH₂-P (aminoethylphosphonate)), 17.0 (2 x CH₃).
³¹P-NMR (500 MHz, MeOD), δ 28.8 (s).
MS (ESI):
m/z +1: 479.0
m/z -1: 477.1

### Example 9: Uridine-5'-carboxylic acid [dimethylphosphono,(methoxycarbonyl)-methylcarbamoyl-methyl]amide

Commercially available N-benzyloxycarbonyl-α-phosphonoglycine (11 mmol, 3600 mg) is dissolved in 20 ml of dry methanol and hydrogenated for 1 hour at 3 atm H₂ (rt) with 1 g of Pd/C. The suspension is filtered, the catalyst washed with methanol (2 x 5 ml) and the filtrate directly used in the next step.

In a dry vessel, N-tert-butyloxycarbonylglycine (10 mmol, 1750 mg) is dissolved in 10 ml of dry THF and cooled to -25 °C. Subsequently, N-methylmorpholine (10 mmol, 1010 mg) and isobutyl chloroformiate (10 mmol, 1360 mg) are sequentially added under vigorous stirring. Immediately after the formation of a white precipitate (N-methylmorpholine hydrochloride) the solution of α-phosphonoglycine in methanol (30 ml), pre-cooled on ice, is added. The resulting mixture is allowed to warm to ambient temperature. After three hours, the volatiles are removed by rotary evaporation at 40 °C, the residue is dissolved in 10 ml of water and adjusted to pH 1 (10% aq. NaHSO₄ solution) and extracted with ethyl acetate (3 x 50 ml). The combined organic layers are washed with satured aq. Na₂CO₃ solution (3 x 20 ml) and subsequently with water (3 x 20 ml), dried over Na₂SO₄, and evaporated to dryness.

The residue (boc-protected amide) is dissolved in 8 ml of dry 4N HCl-dioxane solution and stirred for two hours at ambient temperature. N-(Aminomethylcarbonyl)-α-dimethylphosphonoglycine methyl ester hydrochloride is precipitated by addition of 50 ml of diethyl ether, filtered off and thoroughly washed with diethyl ether.
Yield over three steps: 1650 mg, 65 %, white crystals.

Under an atmosphere of argon, 2 ',3'-anisylideneuridine- 5'-carboxylic acid (1 mmol, 376 mg), HCTU^{®} (1.1 mmol, 455 mg) and 1-hydroxybenzotriazole (1.1 mmol, 149 mg) are dissolved in 2 ml of dry DMF at ambient temperature. Diisopropylethylamine (1.1 mmol, 143 mg) and, after one minute, N-(aminomethylcarbonyl)-α-dimethylphosphonoglycine methyl ester hydrochloride (2 mmol, 550 mg), dissolved in a mixture of dry DMF (2 ml) and diisopropylethylamine (0.5 ml), are added sequentially via a syringe to the solution. Vigorous stirring is continued for 24 hours at ambient temperature. The volatiles are removed in vacuo at 40 °C and the residue is purified by silica gel column chromatography using dichloromethane/methanol (40:1). The product is isolated by rotary evaporation at 40 °C and recrystallized from diethyl ether.

Deprotection of the ribose moiety is performed by stirring 2',3'-anisylideneuridine-5'-amide (100 mg) in a mixture of dichloromethane (3 ml), trifluoroacetic acid (0.15 ml) and water (one drop) at ambient temperature. After two hours, the crude product is precipitated by addition of diethyl ether (50 ml), filtered off, dissolved in 7 ml of water : methanol (75 : 25) and purified by RP-HPLC using a gradient of water : methanol from 75 : 25 to water : methanol 0 : 100. Uridine-5'-carboxylic acid [dimethylphosphono,(methyoxycarbonyl)methylcarbamoyl-methyl] amide is isolated as a mixture of two stereoisomeres by lyophilisation.

Yield over two steps: 260 mg (53%), white amorphous powder
¹H-NMR (500 MHz, MeOD), δ 8.10 (2 x d, 1H, ³J = 7.85 Hz, H-6), 5.96 (2 x d, 1H, ³J = 5.95 Hz, H-1'), 5.77 (2 x d, 1H, ³J = 7.85 Hz, H-5), 4.50-4.40 (m, 3H, H-2', H-3' and H-4'), 4.18 - 3.94 (AB-system with A d and B d, 2H, ²J = 17.00 Hz, N-CH₂ (carbamoylmethylamine)), 3.97 - 3.85 (m, 9H, 3 x OCH₃), N-CH (a-phosphonoglycine) not determinable, under solvent peak at 3.35 ppm.
¹³C-NMR (500 MHz, MeOD), δ 173.3 (C=O), 171.4 (C=O), 168.1 (C=O), 166.4 (C-4), 153.0 (C-2), 144.6 (C-6), 103.5 (C-5), 93.3 (C-1'), 85.6 (H-4'), 74.9 (H-2'), 74.2 (H-3'), 55.3 (3 x OCH₃), N-CH (a-phosphonoglycine) not determinable, under solvent peak at 49 ppm, 43.1 (N-CH₂ (carbamoylmethylamine))
³¹P-NMR (500 MHz, MeOD), δ 18.1.
MS (ESI):
m/z +1: 495.0
m/z -1: 493.3

### Example 10: Uridine-5'-carboxylic acid [bis(diethylphosphono)methylcarbamoylmethyl]amide

In a dry vessel, N-tert-butyloxycarbonyl-glycine (10 mmol, 1750 mg) is dissolved in 10 ml of dry THF and cooled to -25 °C. Subsequently, N-methylmorpholine (10 mmol, 1010 mg) and isobutyl chloroformiate (10 mmol, 1360 mg) are sequentially added under vigorous stirring. Immediately after the formation of a white precipitate (N-methylmorpholine hydrochloride) a solution of bis(diethylphosphono)methyleneamine (11 mmol, 3350 mg) in dry THF (10 ml) is added. The resulting mixture is allowed to warm to ambient temperature. After three hours, the volatiles are removed by rotary evaporation at 40 °C, the residue is dissolved in 10 ml of water and adjusted to pH 1 (10% aq. NaHSO₄ solution) and extracted with ethyl acetate (3 x 50 ml). The combined organic layers are washed with satured aq. Na₂CO₃ solution (3 x 20 ml) and subsequently with water (3 x 20 ml), dried over Na₂SO₄, and evaporated to dryness.

The residue (boc-protected amide) is dissolved in 8 ml of dry 4N HCl-dioxane solution and stirred for two hours at ambient temperature. Aminomethylcarboxamidomethylbis-(phosphonic acid diethyl ester) hydrochloride is precipitated by addition of 50 ml of diethyl ether, filtered off and thoroughly washed with diethyl ether.
Yield over two steps: 3100 mg, 76 %, clay.

Under an atmosphere of argon, 2' ,3' -anisylideneuridine-5'-carboxylic acid (1 mmol, 376 mg), HCTU^{®} (1.1 mmol, 455 mg) and 1-hydroxybenzotriazole (1.1 mmol, 149 mg) are dissolved in 2 ml of dry DMF at ambient temperature. Diisopropylethylamine (1.1 mmol, 143 mg) and, after one minute, aminomethylcarboxamidomethyl-bis(phosphonic acid diethyl ester) hydrochloride (2 mmol, 788 mg), dissolved in a mixture of dry DMF (2 ml) and diisopropylethylamine (0.5 ml), are added sequentially via a syringe to the solution. Vigorous stirring is continued for 24 hours at ambient temperature. The volatiles are removed in vacuo at 40 °C and the residue is purified by silica gel column chromatography using dichloromethane/methanol (40:1). The product is isolated by rotary evaporation at 40 °C and recrystallized from diethyl ether. Deprotection of the ribose moiety is performed by stirring 2',3'-anisylideneadenosine-5'-amide (100 mg) in a mixture of dichloromethane (3 ml), trifluoroacetic acid (0.15 ml) and water (one drop) at ambient temperature. After two hours, the crude product is precipitated by addition of diethyl ether (50 ml), filtered off, dissolved in 7 ml of water/methanol (75 : 25) and purified by RP-HPLC using a gradient of water : methanol from 75 : 25 to water : methanol 0 : 100. Uridine-5' -carboxylic acid [bis(diethylphosphono)methylcarbamoylmethyl]amide is isolated by lyophilisation.
Yield over two steps: 320 mg (50 %), white amorphous powder
¹H-NMR (500 MHz, DMSO-d₆), δ 11.30 (d, 1H, ³J = 1.85 Hz, NH (uracil)), 8.74 (d, 1H, ³J = 9.80 Hz, CONH), 8.51 (t, 1H, ³j = 5.65 Hz, 5'-CONH), 8.23 (d, 1H, ³j = 8.15 Hz, H-6), 5.92 (d, 1H, ³j = 6.90 Hz, H-1'), 5.62 (dd, 1H, ³j = 7.90 Hz and ⁴J = 2.20 Hz, H-5), 5.52 (br s, 2H, 2 x OH), 4.82 (td, 1H, ³j = 9.75 Hz and ²J_{H,P} = 22.35 Hz, PPNCH (aminomethylbisphosphonate)), 4.35 (d, 1H, ³J = 1.90 Hz, H-4'), 4.20 - 3.99 (br s, 10H, 4 x OCH₂, H-2' and H-3'), 3.85 (AB-system with A dd and B dd, 1H, ³J = 5.70 Hz and ²J = 17.30 Hz, N-CH₂ (carbamoylmethylamine)), 1.22 (br s, 12H, 4 x CH₃).
¹³C-NMR (500 MHz, DMSO-d₆), δ 170.5 (C=O), 168.7 (C=O), 163.2 (C-4), 151.2 (C-2), 141.4 (C-6), 102.2 (C-5), 87.8 (C-1'-), 83.2 (C-4'), 73.9 (C-2'), 72.2 (C-3'), 63.1 (O-CH₂), 43.5 (t, partially under solvent peak, ¹J_{C,P} = 581.90 Hz, PPNCH (aminomethylbisphosphonate)), N-CH₂ (carbamoylmethylamine) under solvent peak at 42, 16.3 (CH₃).
³¹P-NMR (500 MHz, DMSO-d₆), 15.8.

### Example 11: Uridine-5'-carboxylic acid [2-(bis(diethylphosphono)methylcarbamoyl)ethyl]amide

In a dry vessel, N-tert-butyloxycarbonyl-β-alanine (10 mmol, 1890 mg) is dissolved in 10 ml of dry THF and cooled to -25 °C. Subsequently, N-methylmorpholine (10 mmol, 1010 mg) and isobutyl chloroformiate (10 mmol, 1360 mg) are sequentially added under vigorous stirring. Immediately after the formation of a white precipitate (N-methylmorpholine hydrochloride) a solution of bis(diethylphosphono)methyleneamine (11 mmol, 3350 mg) in dry THF (10 ml) is added. The resulting mixture is allowed to warm to ambient temperature. After three hours, the volatiles are removed by rotary evaporation at 40 °C, the residue is dissolved in 10 ml of water and adjusted to pH 1 (10% aq. NaHSO₄ solution) and extracted with ethyl acetate (3 x 50 ml). The combined organic layers are washed with satured aq. Na₂CO₃ solution (3 x 20 ml) and subsequently with water (3 x 20 ml), dried over Na₂SO₄, and evaporated to dryness.

The residue (boc-protected amide) is dissolved in 8 ml of dry 4N HCl-dioxane solution and stirred for two hours at ambient temperature. 2-Aminoethylcarboxamidomethyl-bis(phosphonic acid diethyl ester) hydrochloride is precipitated by addition of 50 ml of diethyl ether, filtered off and thoroughly washed with diethyl ether.
Yield over two steps: 3100 mg, 76 %, clay.
Under an atmosphere of argon, 2',3'-anisylideneadenosine-5'-carboxylic acid (1 mmol, 376 mg), PyBOP^{®} (1.1 mmol, 572 mg) and 1-hydroxybenzotriazole (1.1 mmol, 149 mg) are dissolved in 2 ml of dry DMF at ambient temperature. Diisopropylethylamine (1.1 mmol, 143 mg) and, after one minute, 2-aminoethylcarboxamidomethyl-bis(phosphonic acid diethyl ester) hydrochloride (2 mmol, 816 mg), dissolved in a mixture of dry DMF (2 ml) and diisopropylethylamine (0.5 ml), are added sequentially via a syringe to the solution. Vigorous stirring is continued for 24 hours at ambient temperature. The volatiles are removed in vacuo at 40 °C and the residue is purified by silica gel column chromatography using dichloromethane/methanol (40:1). The product is isolated by rotary evaporation at 40 °C and recrystallized from diethyl ether. Deprotection of the ribose moiety is performed by stirring 2',3'-anisylideneadenosine-5'-amide (100 mg) in a mixture of dichloromethane (3 ml), trifluoroacetic acid (0.15 ml) and water (one drop) at ambient temperature. After two hours, the crude product is precipitated by addition of diethyl ether (50 ml), filtered off, dissolved in 7 ml of water/methanol (75 : 25) and purified by RP-HPLC using a gradient of water : methanol from 75 : 25 to water : methanol 0 : 100. Uridine-5'-carboxylic acid [2-(bis(diethylphosphono)methylcarbamoyl)-ethyl]amide is isolated by lyophilisation.
Yield over two steps: 320 mg (50 %), white amorphous powder
¹H-NMR (500 MHz, DMSO-d₆), δ 11.30 (d, 1H, ⁴J = 2.25 Hz, NH (uracil)), 8.74 (d, 1H, ³J = 10.05 Hz, CONH (β-Ala)), 8.28 (t, 1H, ³J = 5.65 Hz, 5'-CONH), 8.23 (d, 1H, ³J = 8.20 Hz, H-6), 5.88 (d, 1H, ³J = 6.30 Hz, H-1'), 5.69 (dd, 1H, ³J = 7.90 Hz and ⁴J = 2.20 Hz, H-5'), 5.48 (br s, 2H, 2'-OH and 3'-OH), 4.86 (dt, 1H, ³J = 10.1 Hz and ²J_{P,H} = 22.70 Hz (aminomethylbisphosphonate)), 4.23 (d, 1H, ³J = 2.20 Hz, H-4'), 4.16 (pseudo-t, 1H, ³J = 4.72 Hz and ³J = 6.60 Hz, H-2'), 4.02 (br s, 8H, 4 x O-CH₂), 3.99 (pseudo-q, 1H, ³J = 2.20 Hz and ³J = 2.20 Hz and ³J = 2.50 Hz, H-3'), 3.40 - 3.20 (N-CH₂ (carbamoylethylamine), not determinable, covered by water from solvent), 2.44 (t, 2H, ³J = 6.95 Hz, O=C-CH₂ (carbamoylethylamine)), 1.21 (m, 12H, 4 x CH₃).
¹³C-NMR (500 MHz, DMSO-d₆), δ 172.1 (C=O), 170.2 (C=O), 163.2 (C-4), 151.1 (C-2), 143.5 (C-6), 102.2 (C-5), 88.0 (C-1'), 83.2 (C-4'), 73.1 (C-2'), 73.0 (C-3'), 63.0 and 62.8 (OCH₂), 43.3 (t, partially covered by solvent peak, ¹J = 579.90 Hz, PPNCH (aminomethylbisphosphonate)), 35.4 (N-CH₂ (carbamoylethylamine)), 34.4 (O=C-CH₂ carbamoylethylamine)), 16.4 and 16.3 (CH₃).
³¹P-NMR (500 MHz, DMSO-d₆), 15.2.

### Example 12: Uridine-5'carboxylic acid [3-(bis(diethylphosphono)methylcarbamoyl)propyl]amide

In a dry vessel, N-tert-butyloxycarbonyl-γ-aminobutyric acid (10 mmol, 2030 mg) is dissolved in 10 ml of dry THF and cooled to -25 °C. Subsequently, N-methylmorpholine (10 mmol, 1010 mg) and isobutyl chloroformiate (10 mmol, 1360 mg) are sequentially added under vigorous stirring. Immediately after the formation of a white precipitate (N-methylmorpholine hydrochloride) a solution of of bis(diethylphosphono)methyleneamine (11 mmol, 3350 mg) in dry THF (10 ml) is added.. The resulting mixture is allowed to warm to ambient temperature. After three hours, the volatiles are removed by rotary evaporation at 40 °C, the residue is dissolved in 10 ml of water and adjusted to pH 1 (10% aq. NaHSO₄ solution) and extracted with ethyl acetate (3 x 50 ml). The combined organic layers are washed with satured aq. Na₂CO₃ solution (3 x 20 ml) and subsequently with water (3 x 20 ml), dried over Na₂SO₄, and evaporated to dryness.

The residue (boc-protected amide) is dissolved in 8 ml of dry 4N HCl-dioxane solution and stirred for two hours at ambient temperature. 3-Aminopropylcarboxamidomethyl-bis(phosphonic acid diethyl ester) hydrochloride is precipitated by addition of 50 ml of diethyl ether, filtered off and thoroughly washed with diethyl ether.
Yield over two steps: 3400 mg, 80 %.
Under an atmosphere of argon, 2' ,3' -anisylideneuridine- 5' -carboxylic acid (1 mmol, 376 mg), PyBOP^{®} (1.1 mmol, 572 mg) and 1-hydroxybenzotriazole (1.1 mmol, 149 mg) are dissolved in 2 ml of dry DMF at ambient temperature. Diisopropylethylamine (1.1 mmol, 143 mg) and, after one minute, 3-aminopropylcarboxamidomethyl-bis(phosphonic acid diethyl ester) hydrochloride (2 mmol, 844 mg), dissolved in a mixture of dry DMF (2 ml) and diisopropylethylamine (0.5 ml), are added sequentially via a syringe to the solution. Vigorous stirring is continued for 24 hours at ambient temperature. The volatiles are removed in vacuo at 40 °C and the residue is purified by silica gel column chromatography using dichloromethane/methanol (40:1). The product is isolated by rotary evaporation at 40 °C and recrystallized from diethyl ether. Deprotection of the ribose moiety is performed by stirring 2',3'-anisylideneadenosine-5'-amide (100 mg) in a mixture of dichloromethane (3 ml), trifluoroacetic acid (0.15 ml) and water (one drop) at ambient temperature. After two hours, the crude product is precipitated by addition of diethyl ether (50 ml), filtered off, dissolved in 7 ml of water/methanol (75 : 25) and purified by RP-HPLC using a gradient of water : methanol from 75 : 25 to water : methanol 0 : 100. Uridine-5'-carboxylic acid [3-(bis(diethylphosphono)methylcarbamoyl)-propyl]amide is isolated by lyophilization.
Yield over two steps: 380 g (58 %), white amorphous powder
¹H-NMR (500 MHz, DMSO-d₆), δ 11.30 (d, 1H, ³J = 1.85 Hz, NH (uracil)), 8.63 (d, 1H, ³J = 9.75 Hz, CONH), 8.31 (t, 1H, ³J = 5.65 Hz, 5'-CONH), 8.27 (d, 1H, ³J = 8.15 Hz, H-6), 5.86 (d, 1H, ³J = 6.30 Hz, H-1'), 5.62 (dd, 1H, ³J = 8.15 Hz and ⁴J = 2.20 Hz, H-5), 5.50 (br s, 2H, 2 x OH), 4.87 (td, 1H, ³J = 10.10 Hz and ²J_{H,P} = 23.00 Hz (aminomethylbisphosphonate)), 4.25 (d, 1H, ³J = 2.50 Hz, H-4'), 4.17 (pseudo-t, 1H, ³J = 4.75 Hz and ³J = 5.95 Hz, H-2') 4.08 - 4.02 (m, 8H, 4 x O-CH₂), 3.98 (dd, 1H, ³J = 2.50 Hz and ³J = 4.40 Hz, H-3'), 3.08 (dt, 2H, ³J = 5.70 Hz and ³J = 7.50 Hz, N-CH₂ (carbamoylpropylamine)), 2.23 (t, 2H, ³J = 7.25 Hz (O=C-CH₂ (carbamoylpropylamine)), 1.65 (tt, 2H, ³J = 7.25 Hz, CH₂ (carbamoylpropylamine)), 1.22 (br s, 12H, 4 x CH₃).
¹³C-NMR (500 MHz, DMSO-d₆), δ 171.8 (C=O), 170.0 (C=O), 163.2 (C-4), 151.2 (C-2), 141.4 (C-6), 102.1 (C-5), 88.3 (C-1'), 83.3 (C-4'), 73.2 (C-2'), 73.1 (C-3'), 62.9 (O-CH₂), 43.5 (t, ¹J_{C,P} = 589.80 Hz, PPNCH (aminomethylbisphosphonate)), 38.3 (N-CH₂ (carbamoylpropylamine)), 32.3 (O=C-CH₂ (carbamoylpropylamine)), 25.5 (CH₂ (carbamoylpropylamine)), 16.3 (CH₃).
³¹P-NMR (500 MHz, DMSO-d₆), 15.6.

### Example 13: Adenosine-5'-carboxylic acid [p-(diethylphosphonomethyl)phenylcarbamoylmethyl]amide

In a dry vessel, N-tert-butyloxycarbonylglycine (10 mmol, 1750 mg) is dissolved in 10 ml of dry THF and cooled to -25 °C. Subsequently, N-methylmorpholine (10 mmol, 1010 mg) and isobutyl chloroformiate (10 mmol, 1360 mg) are sequentially added under vigorous stirring. Immediately after the formation of a white precipitate (N-methylmorpholine hydrochloride) a solution of aminobenzylphosphonic acid diethyl ester (11 mmol, 2673 mg) in dry THF (10 ml) is added. The resulting mixture is allowed to warm to ambient temperature. After three hours, the volatiles are removed by rotary evaporation at 40 °C, the residue is dissolved in 10 ml of water and adjusted to pH 1 (10 % aq. NaHSO₄ solution) and extracted with ethyl acetate (3 x 50 ml). The combined organic layers are washed with saturated aq. Na₂CO₃ solution (3 x 20 ml) and subsequently with water (3 x 20 ml), dried over Na₂SO₄, and evaporated to dryness.

The residue (boc-protected amide) is dissolved in 8 ml of dry 4N HCl-dioxane solution and stirred for two hours at ambient temperature. p-(Aminomethylcarboxamido)benzylphosphonic acid diethyl ester hydrochloride is precipitated by addition of 50 ml of diethyl ether, filtered off and thoroughly washed with diethyl ether.
Yield over two steps: 3100 mg, 92 %, white crystals.
Under an atmosphere of argon, 2',3'-anisylideneadenosine-5'-carboxylic acid (1 mmol, 399 mg), HCTU^{®} (1.1 mmol, 455 mg) and 1-hydroxybenzotriazole (1.1 mmol, 149 mg) are dissolved in 2 ml of dry DMF at ambient temperature. Diisopropylethylamine (1.1 mmol, 143 mg) and, after one minute, p-(aminomethylcarboxamido)benzylphosphonic acid diethyl ester hydrochloride (2 mmol, 672 mg), dissolved in a mixture of dry DMF (2 ml) and diisopropylethylamine (0.5 ml), are added sequentially via a syringe to the solution. Vigorous stirring is continued for 24 hours at ambient temperature. The volatiles are removed in vacuo at 40 °C and the residue is purified by silica gel column chromatography using dichloromethane : methanol (40 : 1). The product is isolated by rotary evaporation at 40 °C and recrystallized from diethyl ether.

Deprotection of the ribose moiety is performed by stirring 2',3'-anisyiideneadenosine-5'-amide (100 mg) in a mixture of dichloromethane (3 ml), trifluoroacetic acid (0.15 ml) and water (one drop) at ambient temperature. After two hours, the crude product is precipitated by the addition of diethyl ether (50 ml), filtered off, dissolved in 7 ml of water : methanol (75 : 25) and purified by RP-HPLC using a gradient of water : methanol from 75 : 25 to water : methanol 0 : 100. Adenosine-5'-carboxylic acid [p-(diethylphosphonomethyl)-phenylcarbamoylmethyl]amide is isolated by lyophilization.
Yield over two steps: 310 mg (68 %), white amorphous powder
¹H-NMR (500 MHz, MeOD), δ 8.42 (s, 1H, H-2), 8.24 (s, 1H, H-8), 7.57 (d, 2H, ³J = 8.20 Hz, 2 x CH, (aromate, p-aminobenzylphosphonate)), 7.30 (dd, 2H, ³J = 8.80 Hz and ⁴J = 2.50 Hz, 2 x CH (aromate, p-aminobenzylphosphonate)), 6.14 (d, 1H, ³J = 7.90 Hz, H-1 '), 4.91 (pseudo-q, partly covered by solvent, 1H, ³J = 7.55 Hz and ³J = 4.75 Hz, H-2'), 4.62 (s, 1H, H-4'), 4.49 (dd, 1H, ³J = 4.75 and ³J = 1.60 Hz, H-3 '), 4.29 - 4.10 (AB-system with A d and B d, 2H, ²J = 16.40 Hz, N-CH₂ (carbamoylmethylamine)), 4.09 - 4.04 (m, 4H, 2 x O-CH₂), 3.24 (d, 2H, ²J_{H,P} = 21.45 Hz, CH₂-P (p-aminobenzylphosphonate)), 1.29 (t, 6H, 2 x CH₃).
¹³C-NMR (500 MHz, MeOD), δ 173.4 (C=O), 169.5 (C=O), 157.9 (C-6), 154.2 (C-2), 150.6 (C-4), 142.8 (C-8), 138.9 (C-NH (aromate, p-aminobenzylphosphonate, para-position)), 131.7 (2 x CH (aromate, p-aminobenzylphosphonate, ortho-position)), 128.7 (d, ²J_{C,P} = 37.7 Hz, C-CH₂-P (aromate, p-aminobenzylphosphonate)), 121.5 (2 x CH (aromate, p-aminobenzylphosphonate, meta-position)), 121.3 (C-5), 90.6 (C-1'), 86.7 (C-4'), 75.5 (C-2'), 73.9 (C-3'), 64.0 (2 x OCH₂), 44.0 (N-CH₂ (carbamoylmethylamine)), 33.4 (d, ¹J_{C,P} = 550.2 Hz, CH₂-P (p-aminobenzylphosphonate)), 17.0 und 16.9 (2 x CH₃).
³¹P-NMR (500 MHz, MeOD), δ 28.8 (s).
MS (ESI):
m/z +1: 564.3
m/z -1: 562.3

### Example 14: Adenosine-5'-carboxylic acid [2-(p-(diethylphosphonomethyl)-phenylcarbamoyl)ethyl]amide

In a dry vessel, N-tert-butyloxycarbonyl-β-alanine (10 mmol, 1890 mg) is dissolved in 10 ml of dry THF and cooled to -25 °C. Subsequently, N-methylmorpholine (10 mmol, 1010 mg) and isobutyl chloroformiate (10 mmol, 1360 mg) are sequentially added under vigorous stirring. Immediately after the formation of a white precipitate (N-methylmorpholine hydrochloride) a solution of aminobenzylphosphonic acid diethyl ester (11 mmol, 2673 mg) in dry THF (10 ml) is added. The resulting mixture is allowed to warm to ambient temperature. After three hours, the volatiles are removed by rotary evaporation at 40 °C, the residue is dissolved in 10 ml of water and adjusted to pH 1 (10% aq. NaHSO₄ solution) and extracted with ethyl acetate (3 x 50 ml). The combined organic layers are washed with satured aq. Na₂CO₃ solution (3 x 20 ml) and subsequently with water (3 x 20 ml), dried over Na₂SO₄, and evaporated to dryness.

The residue (boc-protected amide) is dissolved in 8 ml of dry 4N HCI-dioxane solution and stirred for two hours at ambient temperature. p-(2-Aminoethylcarboxamido)benzylphosphonic acid diethyl ester hydrochloride is precipitated by addition of 50 ml of diethyl ether, filtered off and thoroughly washed with diethyl ether.
Yield over two steps: 3300 mg, 94 %, white crystals.
Under an atmosphere of argon, 2',3'-anisylideneadenosine-5'-carboxylic acid (1 mmol, 399 mg), HBTU^{®} (1.1 mmol, 428 mg) and 1-hydroxybenzotriazole (1.1 mmol, 149 mg) are dissolved in 2 ml of dry DMF at ambient temperature. Diisopropylethylamine (1.1 mmol, 143 mg) and, after one minute, p-(2-aminoethylcarboxamido)benzylphosphonic acid diethyl ester hydrochloride (2 mmol, 700 mg), dissolved in a mixture of dry DMF (2 ml) and diisopropylethylamine (0.5 ml), are added sequentially via a syringe to the solution. Vigorous stirring is continued for 24 hours at ambient temperature. The volatiles are removed in vacuo at 40 °C and the residue is purified by silica gel column chromatography using dichloromethane/methanol (40:1). The product is isolated by rotary evaporation at 40 °C and recrystallized from diethyl ether. Deprotection of the ribose moiety is performed by stirring 2',3'-anisylideneadenosine-5'-amide (100 mg) in a mixture of dichloromethane (3 ml), trifluoroacetic acid (0.15 ml) and water (one drop) at ambient temperature. After two hours, the crude product is precipitated by addition of diethyl ether (50 ml), filtered off, dissolved in 7 ml of water/methanol (75 : 25) and purified by RP-HPLC using a gradient of water : methanol from 75 : 25 to water : methanol 0:100. Adenosine-5'-carboxylic acid [2-(p-(diethylphosphonomethyl)phenylcarbamoyl)ethyl]amide is isolated by lyophilisation.
Yield over two steps: 350 mg (71 %), white amorphous powder
¹H-NMR (500 MHz, D₂O) δ 8.08 (s, 1H, H-2), 7.95 (s, 1H, H-8), 7.57 (d, 2H, ³J = 8.20 Hz, 2 x CH, (aromate, p-aminobenzylphosphonate)), 7.30 (dd, 2H, ³J = 8.80 Hz and ⁴J = 2.50 Hz, 2 x CH (aromate p-aminobenzylphosphonate)), 6.14 (d, 1H, ³J = 7.90 Hz, H-1'), 4.91 (pseudo-q, partly covered by solvent, 1H, ³J = 7.55 Hz and ³J = 4.75 Hz, H-2'), 4.62 (s, 1H, H-4'), 4.49 (dd, 1H, ³J = 4.75 and ³J = 1.60 Hz, H-3'), 4.09 - 4.04 (m, 4H, 2 x O-CH₂), 3.67 - 3.58 (AA'XX'-system with A ddd and A' ddd, 2H, ³J_{A,X} = 5.35 Hz and ³J_{A',X} = 5.65 Hz and ³J_{A,X'} = 5.05 Hz and ³J_{A',X'} = 5.70 Hz and ²J_{A,B} = 13.90 Hz, N-CH₂ (carbamoylethylamine)), 3.24 (d, 2H, ²J_{H,P} = 21.45 Hz, CH₂-P (p-aminobenzylphosphonate)), 2.59 - 2.49 (AA'XX'-system with X ddd and X' ddd, 2H, ³J_{X,A} = 5.35 Hz and ³J_{X,A'} = 6.45 Hz and ³J_{X',A} = 5.05 Hz and ³J_{X',A'} = 6.60 Hz and ²J_{X,X'} = 13.85 Hz, O=C-CH₂ (carbamoylethylamine)), 1.29 (t, 6H, 2 x CH₃).
¹³C-NMR (500 MHz, D₂O) δ 174.9 (C=O), 174.4 (C=O), 157.9 (C-6), 155.3 (C-2), 150.7 (C-4), 143.9 (C-8), 138.8 (C-NH (aromate, p-aminobenzylphosphonate, para-position)), 133.1 (2 x CH (aromate, p-aminobenzylphosphonate, ortho-position)), 129.6 (d, ²J_{C,P} = 37.7 Hz, C-CH₂P (aromate, p-aminobenzylphosphonate)), 123.1 (2 x CH (aromate, p-aminobenzylphosphonate meta-position)), 121.9 (C-5), 91.3 (C-1'), 87.4 (C-4'), 75.8 (C-2'), 74.7 (C-3'), 64.0 (2 x O-CH₂), 39.6 (N-CH₂ (carbamoylethylamine)), 39.1 (O=C-CH₂ (carbamoylethylamine)), 34.1 (d, ¹J_{C,P} = 550.2 Hz, CH₂-P (p-aminobenzylphosphonate)), 18.3 (2 x CH₃).
³¹ P-NMR (500 MHz, D₂O) δ 30.0 (s).
MS (ESI):
m/z +1: 578.2
m/z-1: 576.3

### Example 15: Adenosine-5'-carboxylic acid [3-(p-(diethylphosphonomethyl)-phenylcarbamoyl)propyl]amide

In a dry vessel, N-tert-butyloxycarbonyl-y-aminobutyric acid (10 mmol, 2030 mg) is dissolved in 10 ml of dry THF and cooled to -25 °C. Subsequently, N-methylmorpholine (10 mmol, 1010 mg) and isobutyl chloroformiate (10 mmol, 1360 mg) are sequentially added under vigorous stirring. Immediately after the formation of a white precipitate (N-methylmorpholine hydrochloride) a solution of aminobenzylphosphonic acid diethyl ester (11 mmol, 2673 mg) in dry THF (10 ml) is added. The resulting mixture is allowed to warm to ambient temperature. After three hours, the volatiles are removed by rotary evaporation at 40 °C, the residue is dissolved in 10 ml of water and adjusted to pH 1 (10% aq. NaHSO₄ solution) and extracted with ethyl acetate (3 x 50 ml). The combined organic layers are washed with satured aq. Na₂CO₃ solution (3 x 20 ml) and subsequently with water (3 x 20 ml), dried over Na₂SO₄, and evaporated to dryness.

The residue (boc-protected amide) is dissolved in 8 ml of dry 4N HCl-dioxane solution and stirred for two hours at ambient temperature. p-(3-Aminopropylcarboxamido) benzylphosphonic acid diethyl ester hydrochloride is precipitated by addition of 50 ml of diethyl ether, filtered off and thoroughly washed with diethyl ether.
Yield over two steps: 3300 mg, 94 %, white crystals.
Under an atmosphere of argon, 2',3'-anisylideneadenosine-5'-carboxylic acid (1 mmol, 399 mg), HBTU^{®} (1.1 mmol, 428 mg) and 1-hydroxybenzotriazole (1.1 mmol, 149 mg) are dissolved in 2 ml of dry DMF at ambient temperature. Diisopropylethylamine (1.1 mmol, 143 mg) and, after one minute, p-(3-aminopropylcarboxamido)benzylphosphonic acid diethyl ester hydrochloride (2 mmol, 728 mg), dissolved in a mixture of dry DMF (2 ml) and diisopropylethylamine (0.5 ml), are added sequentially via a syringe to the solution. Vigorous stirring is continued for 24 hours at ambient temperature. The volatiles are removed in vacuo at 40 °C and the residue is purified by silica gel column chromatography using dichloromethane/methanol (40:1). The product is isolated by rotary evaporation at 40 °C and recrystallized from diethyl ether. Deprotection of the ribose moiety is performed by stirring 2',3'-anisylideneadenosine-5'-amide (100 mg) in a mixture of dichloromethane (3 ml), trifluoroacetic acid (0.15 ml) and water (one drop) at ambient temperature. After two hours, the crude product is precipitated by addition of diethyl ether (50 ml), filtered off, dissolved in 7 ml of water/methanol (75 : 25) and purified by RP-HPLC using a gradient of water : methanol from 75 : 25 to water : methanol 0 : 100. Adenosine-5'-carboxylic acid [3-(p-(diethylphosphonomethyl)phenylcarbamoyl)propyl]amide is isolated by lyophilisation.
Yield over two steps: 340 mg (70 %), white amorphous powder
¹H-NMR (500 MHz, DMSO-d₆), δ 9.84 (br s, 1H, 5'-CONH), 9.05 (t, 2H, ³J = 5.70 Hz, CONH (carbamoylpropylamine)), 8.36 (s, 1H, H-2), 8.23 (s, 1H, H-8), 7.48 (d, 2H, ³J = 8.55 Hz, 2 x CH, (aromate, p-aminobenzylphosphonate)), 7.38 (br s, 2H, NH₂ (adenine)), 7.16 (dd, 2H, ³J = 8.85 Hz and ⁴J = 2.20 Hz, 2 x CH (aromate, p-aminobenzylphosphonate)), 5.95 (d, 1H, ³J = 7.60 Hz, H-1'), 5.72 (br d, 1H, ³J = 2.50 Hz, 2'-OH), 5.51 (br d, 1H, ³J = 4.40 Hz, 3'-OH), 4.61 (pseudo-q, 1H, ³J = 7.55 Hz and ³J = 4.75 Hz, H-2'), 4.32 (d, 1H, ³J = 1.60 Hz, H-4'), 4.14 (br s, 1H, H-3'), 3.95 - 3.89 (m, 4H, 2 x O-CH₂), 3.26 (dt, 2H, ³J = 7.25 Hz and ³J = 5.70 Hz, N-CH₂ (carbamoylpropylamine)), 3.12 (d, 2H, ²J_{H,P} = 21.10 Hz, CH₂-P (p-aminobenzylphosphonate)), 2.32 (t, 2H, ³J = 7.25 Hz, O=C-CH₂ (carbamoylpropylamine)), 1.79 (tt, 2H, ³J = 7.25 Hz, CH₂ (carbamoylpropylamine)), 1.29 (t, 6H, 2 x CH₃).
¹³C-NMR (500 MHz, DMSO-d₆), δ 170.8 (C=O), 169.6 (C=O), 156.5 (C-6), 152.6 (C-2), 148.9 (C-4), 140.9 (C-8), 137.9 (C-NH (aromate, p-aminobenzylphosphonate, para-position)), 130.1 (2 x CH (aromate, p-aminobenzylphosphonate, ortho-position)), 126.7 (d, ²J_{C,P} = 37.7 Hz, C-CH₂P (aromate, p-aminobenzylphosphonate)), 119.8 (2 x CH (aromate, p-aminobenzylphosphonate, meta-position)), 119.1 (C-5), 88.0 (C-1'), 84.9 (C-4'), 73.4 (C-2'), 72.0 (C-3'), 61.5 (2 x O-CH₂), 38.5 (N-CH₂ (carbamoylpropylamine)), 33.8 (O=C-CH₂ (carbamoylpropylamine)), 33.4 (d, ¹J_{C,P} = 550.2 Hz, CH₂-P (p-aminobenzylphosphonate)), 25.3 (CH₂ (carbamoylpropylamine)), 16.4 und 16.3 (2 x CH₃).
³¹P-NMR (500 MHz, DMSO-d₆), δ 27.1 (s).
MS (ESI):
m/z +1: 592.0
m/z-1: 590.3

### Example 16: Adenosine-5'-carboxylic acid [2-(bis(diethylphosphono)methylcarbamoyl)ethyl]amide

In a dry vessel, N-tert-butyloxycarbonyl-β-alanine (10 mmol, 1890 mg) is dissolved in 10 ml of dry THF and cooled to -25 °C. Subsequently, N-methylmorpholine (10 mmol, 1010 mg) and isobutyl chloroformiate (10 mmol, 1360 mg) are sequentially added under vigorous stirring. Immediately after the formation of a white precipitate (N-methylmorpholine hydrochloride) a solution of bis(diethylphosphono)methyleneamine (11 mmol, 3350 mg) in dry THF (10 ml) is added. The resulting mixture is allowed to warm to ambient temperature. After three hours, the volatiles are removed by rotary evaporation at 40 °C, the residue is dissolved in 10 ml of water and adjusted to pH 1 (10% aq. NaHSO₄ solution) and extracted with ethyl acetate (3 x 50 ml). The combined organic layers are washed with satured aq. Na₂CO₃ solution (3 x 20 ml) and subsequently with water (3 x 20 ml), dried over Na₂SO₄, and evaporated to dryness.

The residue (boc-protected amide) is dissolved in 8 ml of dry 4N HCl-dioxane solution and stirred for two hours at ambient temperature. 2-Aminoethylcarboxamidomethyl-bis(phosphonic acid diethyl ester) hydrochloride is precipitated by addition of 50 ml of diethyl ether, filtered off and thoroughly washed with diethyl ether.
Yield over two steps: 3100 mg, 76 %, clay.
Under an atmosphere of argon, 2',3'-anisylideneadenosine-5'-carboxylic acid (1 mmol, 399 mg), PyBOP^{®} (1.1 mmol, 572 mg) and 1-hydroxybenzotriazole (1.1 mmol, 149 mg) are dissolved in 2 ml of dry DMF at ambient temperature. Diisopropylethylamine (1.1 mmol, 143 mg) and, after one minute, 2-aminoethylcarboxamidomethyl-bis(phosphonic acid diethyl ester) hydrochloride (2 mmol, 816 mg), dissolved in a mixture of dry DMF (2 ml) and diisopropylethylamine (0.5 ml), are added sequentially via a syringe to the solution. Vigorous stirring is continued for 24 hours at ambient temperature. The volatiles are removed in vacuo at 40 °C and the residue is purified by silica gel column chromatography using dichloromethane/methanol (40:1). The product is isolated by rotary evaporation at 40 °C and recrystallized from diethyl ether. Deprotection of the ribose moiety is performed by stirring 2',3'-anisylideneadenosine-5'-amide (100 mg) in a mixture of dichloromethane (3 ml), trifluoroacetic acid (0.15 ml) and water (one drop) at ambient temperature. After two hours, the crude product is precipitated by addition of diethyl ether (50 ml), filtered off, dissolved in 7 ml of water/methanol (75 : 25) and purified by RP-HPLC using a gradient of water : methanol from 75 : 25 to water : methanol 0: 100. Adenosine-5'-carboxylic acid [2-(bis(diethylphosphonyl)methylcarbamoyl)ethyl]amide is isolated by lyophilisation.
Yield over two steps: 320 mg (50 %), white amorphous powder
¹H-NMR (500 MHz, MeOD), δ 8.41 (s, 1H, H-2), 8.38 (s, 1H, H-8), 6.06 (d, 1H, ³J = 7.90 Hz, H-1'), 5.10 (t, 1H, ²J_{H,P} = 23.00 Hz, N-CHPP (aminomethylbisphosphonate)), 4.77 (pseudo-q, 1H, ³j = 7.55 Hz and ³J = 4.75 Hz, H-2'), 4.51 (d, 1H, ³J = 1.55 Hz, H-4'), 4.36 (dd, 1H, ³J = 5.05 Hz and ³J = 1.25 Hz, H-3'), 4.23 - 4.07 (m, 8H, 4 x O-CH₂), 3.74 - 3.61 (AA'XX'-system, 2H, ³J_{A,X} = 6.30 Hz and ³J_{A,X'} = 6.95 Hz and ³J_{A',X} = 6.95 Hz and ³J_{A',X'} = 6.30 Hz and ²J_{A,B} = 13.55 Hz, N-CH₂ (carbamoylethylamine)), 2.66 - 2.61 (AA' XX'-system not determinable instead br m, 2H, ³J = 6.30 Hz and ³J = 6.95 Hz and ²J = 15.75 Hz, O=C-CH₂ (carbamoylethylamine)), 1.36 - 1.25 (m, 12H, 4 x CH₃). ¹³C-NMR (500 MHz, MeOD), δ 173.1 (C=O), 172.8 (C=O), 157.9 (C-6), 154.1 (C-2), 150.5 (C-4), 142.8 (C-8), 121.5 (C-5), 90.7 (C-1'), 86.8 (C-4'), 75.3 (C-2'), 73.7 (C-3'), 65.4 (4 x O-CH₂), 47.5 (t, ¹J_{C,P} = 579.9 Hz, N-CHPP (aminomethylbisphosphonate), 36.7 (N-CH₂ (carbamoylethylamine)), 36.2 (O=C-CH₂ (carbamoylethylamine)), 16.9 (4 x CH₃).
³¹P-NMR (500 MHz, MeOD), δ 15.5.
MS (ESI):
m/z +1: 638.0
m/z-1: 636.3

### Example 17: Adenosine-5'carboxylic acid [3-(bis(diethylphosphono)methylcarbamoyl)propyl]amide

In a dry vessel, N-tert-butyloxycarbonyl-y-aminobutyric acid (10 mmol, 2030 mg) is dissolved in 10 ml of dry THF and cooled to -25 °C. Subsequently, N-methylmorpholine (10 mmol, 1010 mg) and isobutyl chloroformiate (10 mmol, 1360 mg) are sequentially added under vigorous stirring. Immediately after the formation of a white precipitate (N-methylmorpholine hydrochloride) a solution of bis(diethylphosphono)methyleneamine (11 mmol, 3350 mg) in dry THF (10 ml) is added. The resulting mixture is allowed to warm to ambient temperature. After three hours, the volatiles are removed by rotary evaporation at 40 °C, the residue is dissolved in 10 ml of water and adjusted to pH 1 (10% aq. NaHSO₄ solution) and extracted with ethyl acetate (3 x 50 ml). The combined organic layers are washed with satured aq. Na₂CO₃ solution (3 x 20 ml) and subsequently with water (3 x 20 ml), dried over Na₂SO₄, and evaporated to dryness.

The residue (boc-protected amide) is dissolved in 8 ml of dry 4N HCl-dioxane solution and stirred for two hours at ambient temperature. 3-Aminopropylcarboxamidomethyl-bis(phosphonic acid diethyl ester) hydrochloride is precipitated by addition of 50 ml of diethyl ether, filtered off and thoroughly washed with diethyl ether.
Yield over two steps: 3400 mg, 80 %, clay.

Under an atmosphere of argon, 2',3'-anisylideneadenosine-5'-carboxylic acid (1 mmol, 399 mg), PyBOP^{®} (1.1 mmol, 572 mg) and 1-hydroxybenzotriazole (1.1 mmol, 149 mg) are dissolved in 2 ml of dry DMF at ambient temperature. Diisopropylethylamine (1.1 mmol, 143 mg) and, after one minute, 3-aminopropylcarboxamidomethyl-bis(phosphonic acid diethylester) hydrochloride (2 mmol, 844 mg), dissolved in a mixture of dry DMF (2 ml) and diisopropylethylamine (0.5 ml), are added sequentially via a syringe to the solution. Vigorous stirring is continued for 24 hours at ambient temperature. The volatiles are removed in vacuo at 40 °C and the residue is purified by silica gel column chromatography using dichloromethane/methanol (40:1). The product is isolated by rotary evaporation at 40 °C and recrystallized from diethyl ether. Deprotection of the ribose moiety is performed by stirring 2',3'-anisylideneadenosine-5'-amide (100 mg) in a mixture of dichloromethane (3 ml), trifluoroacetic acid (0.15 ml) and water (one drop) at ambient temperature. After two hours, the crude product is precipitated by addition of diethyl ether (50 ml), filtered off, dissolved in 7 ml of water/methanol (75 : 25) and purified by RP-HPLC using a gradient of water : methanol from 75 : 25 to water : methanol 0 : 100. Adenosine-5'carboxylic acid [3-(bis(diethylphosphono)methylcarbamoyl)propyl]amide is isolated by lyophilisation.
Yield over two steps: 380 g (58 %), white amorphous powder
¹H-NMR (500 MHz, MeOD), δ 8.36 (s, 1H, H-2), 8.34 (s, 1H, H-8), 6.07 (d, 1H, ³J = 7.85 Hz, H-1'), 5.14 (t, 1H, ²J_{H,P} = 23.00 Hz, N-CHPP (aminomethylbisphosphonate)), 4.79 (dd, 1H, ³J = 7.55 Hz and ³J = 4.70 Hz, H-2'), 4.51 (d, 1H, ³J = 1.25 Hz, H-4'), 4.36 (dd, 1H, ³J = 4.70 Hz and ³J = 1.25 Hz, H-3'), 4.26 - 4.19 (m, 8H, 4 x O-CH₂), 3.42 (t, 2H, ³J = 6.95 Hz, N-CH₂ (carbamoylpropylamine)), 2.41 (t, 2H, ³J = 7.25 Hz, O=C-C_{H2} (carbamoylpropylamine)), 1.94 (tt, 2H, ³J = 7.25 Hz and ³J = 6.90 Hz, CH₂ (carbamoylpropylamine)), 1.36 - 1.25 (m, 12H, 4 x CH₃).
¹³C-NMR (500 MHz, MeOD), δ 175.0 (C=O), 172.7 (C=O), 158.0 (C-6), 154.3 (C-2), 150.5 (C-4), 143.0 (C-8), 121.5 (C-5), 90.9 (C-1'), 86.8 (C-4 '), 75.3 (C-2'), 73.7 (C-3'), 65.3 (4 x O-CH₂), 45.0 (t, ¹J_{C,P}= 596.8 Hz, N-CHPP (aminomethylbisphosphonate), 39.9 (N-CH₂ (carbamoylpropylamine)), 34.0 (O=C-CH₂ (carbamoylpropylamine)), 27.3 (carbamoylpropylamine), 17.0 (4 x CH₃).
³¹P-NMR (500 MHz, MeOD), δ 15.8.
MS (ESI):
m/z +1: 652.3
m/z -1: 650.3

### Example 18: 2,3-Dihydrouridine-5'-carboxylic acid [(asparto)carbamoylethyl]-amide

In a dry vessel, N-tert-butyloxycarbonyl-β-alanine (10 mmol, 1890 mg) is dissolved in 10 ml of dry THF and cooled to -25 °C. Subsequently, N-methylmorpholine (10 mmol, 1010 mg) and isobutyl chloroformiate (10 mmol, 1360 mg) are sequentially added under vigorous stirring. Immediately after the formation of a white precipitate (N-methylmorpholine hydrochloride) a solution of L-aspartic acid dibenzyl ester p-toluene sulfonate (11 mmol, 5330 mg) in 1 M-aq. NaOH-solution (11 ml) is added. The resulting mixture is allowed to warm to ambient temperature. After three hours, THF and other volatiles are removed by rotary evaporation at 40 °C, the residual aqueous mixture is diluted with a small volume of H₂O and adjusted to pH 1 (10% aq. NaHSO₄ solution) and extracted with ethyl acetate (3 x 50 ml). The combined organic layers are washed with satured aq. Na₂CO₃ solution (3 x 20 ml) and subsequently with water (3 x 20 ml), dried over Na₂SO₄, and evaporated to dryness.

The residue (boc-protected amide) is dissolved in 8 ml of dry 4N HCl-dioxane solution and stirred for two hours at ambient temperature. 2-Aminoethylcarboxamidoaspartic acid dibenzyl ester hydrochloride is precipitated by addition of 50 ml of diethyl ether, filtered off and thoroughly washed with diethyl ether.
Yield over two steps: 2600 mg, 68 %, clay.

Under an atmosphere of argon, 2' ,3' -anisylideneuridine-5-carboxylic acid (1 mmol, 376 mg), PyBOP^{®} (1.1 mmol, 572 mg) and 1-hydroxybenzotriazole (1.1 mmol, 149 mg) are dissolved in 2 ml of dry DMF at ambient temperature. Diisopropylethylamine (1.1 mmol, 143 mg) and, after one minute, 2-aminoethylcarboxamidoglutamic acid dibenzyl ester hydrochloride (2 mmol, 840 mg), dissolved in a mixture of dry DMF (2 ml) and diisopropylethylamine (0.5 ml), are added sequentially via a syringe to the solution. Vigorous stirring is continued for 24 hours at ambient temperature. The volatiles are removed in vacuo at 40 °C and the residue is purified by silica gel column chromatography using dichloromethane/methanol (40:1). The product is isolated by rotary evaporation at 40 °C and recrystallized from diethyl ether.

Deprotection of the ribose moiety is performed by stirring 2',3'-anisylideneuridine-5'-amide (100 mg) in a mixture of dichloromethane (3 ml), trifluoroacetic acid (0.15 ml) and water (one drop) at ambient temperature. After two hours, the crude product is precipitated by addition of diethyl ether (50 ml), filtered off, dissolved in 7 ml of water : methanol (75 : 25) and purified by RP-HPLC using a gradient of water : methanol from 75 : 25 to water : methanol 0 : 100. Uridine-5'-carboxylic acid [(dibenzylasparto)carbamoyiethyl]amide is isolated by lyophilisation.
Yield over two steps: 200 mg (32 %), white amorphous powder

Uridine-5'-carboxylic acid[(dibenzylasparto)carbamoylethyl] amide (30 mg, 0.05 mmol) is suspended by sonification in 2 ml of MeOH and water (5:1). Then, the catalyst Pd(OH)₂ (5 mg) is added, the vessel is purged first by argon and then by hydrogen which are applied by means of a balloon. The reaction is performed overnight at ambient temperature and checked by TLC. After 12 hours the catalyst is filtered off and thoroughly washed with methanol and water. The washings are added to the filtrate. The solvent is removed by lyophilisation.
Yield: 19 mg (89 %)
¹H-NMR (500 MHz, D₂O) δ 5.84 (d, 1H, ³J = 6.35 Hz, H-1'), 4.71 (t, 1H, ³J = 5.35 Hz, N-CH (Asp)), 4.37 (d, 1H, ³J = 2.55 Hz, H-4'), 4.35 (dd, 1H, ³J = 5.35 Hz and ³J = 6.00 Hz, H-2'), 4.33 (dd, 1H, ³J = 2.50 Hz and ³J = 5.35 Hz, H-3'), 3.73 (m, 2H, ³J = 6.60 Hz, N-CH₂ (dihydrouracil)), 3.52 and 3.21 (AB-system with A dd and B dd, 2H, ³J= 6.65 Hz and ²J = 14.80 Hz, OCCH₂ (Asp)), 2.91 (t, 2H, ³J = 6.30 Hz, N-CH₂ (carbamoylethylamine)), 2.79 (m, 2H, ³j = 6.30 Hz and ³J = 2.85 Hz, O=C-C_{H2} (dihydrouracil)), 2.56 (t, 2H, ³J = 6.30 Hz, O=C-CH₂ (carbamoylethylamine)).
¹³C-NMR (500 MHz, D₂O) δ 177.6 (C=O), 176.6 (2 x C=O), 176.4 (C=O), 174.3 (C-4), 157.6 (C-2), 91.5 (C-1'), 84.8 (C-4'), 75.4 (C-2'), 72.3 (C-3'), 45.4 (N-CH (Asp)), 40.6 (N-CH₂ (dihydrouracil)), 39.0 (N-CH₂ (carbamoylethylamine)), 38.4 (O=C-CH₂ (dihydrouracil)), 37.5 (O=C-CH₂ (Asp)), 33.0 (O=C-CH₂ (carbamoylethylamine)).

### Example 19: 2,3-Dihydrouridine-5'-carboxylic acid [(glutamo)carbamoylethyl]-amide

In a dry vessel, N-tert-butyloxycarbonyl-β-alanine (10 mmol, 1890 mg) is dissolved in 10 ml of dry THF and cooled to -25 °C. Subsequently, N-methylmorpholine (10 mmol, 1010 mg) and isobutyl chloroformiate (10 mmol, 1360 mg) are sequentially added under vigorous stirring. Immediately after the formation of a white precipitate (N-methylmorpholine hydrochloride) a solution of L-glutamic acid dibenzyl ester hydrochloride (11 mmol, 3883 mg) in 1 M-aq. NaOH-solution (11 ml) is added. The resulting mixture is allowed to warm to ambient temperature. After three hours, THF and other volatiles are removed by rotary evaporation at 40 °C, the residual aqueous mixture is diluted with a small volume of H₂O and adjusted to pH 1 (10% aq. NaHSO₄ solution) and extracted with ethyl acetate (3 x 50 ml). The combined organic layers are washed with satured aq. Na₂CO₃ solution (3 x 20 ml) and subsequently with water (3 x 20 ml), dried over Na₂SO₄, and evaporated to dryness.

The residue (boc-protected amide) is dissolved in 8 ml of dry 4N HCl-dioxane solution and stirred for two hours at ambient temperature. 2-Aminoethylcarboxamidoglutamic acid dibenzyl ester hydrochloride is precipitated by addition of 50 ml of diethyl ether, filtered off and thoroughly washed with diethyl ether.
Yield over two steps: 2800 mg, 65 %, clay.

Under an atmosphere of argon, 2 ,3' -anisylideneuridine-5' -carboxylic acid (1 mmol, 376 mg), PyBOP^{®} (1.1 mmol, 572 mg) and 1-hydroxybenzotriazole (1.1 mmol, 149 mg) are dissolved in 2 ml of dry DMF at ambient temperature. Diisopropylethylamine (1.1 mmol, 143 mg) and, after one minute, 2-aminoethylcarboxamidoglutamic acid dibenzyl ester hydrochloride (2 mmol, 868 mg), dissolved in a mixture of dry DMF (2 ml) and diisopropylethylamine (0.5 ml), are added sequentially via a syringe to the solution. Vigorous stirring is continued for 24 hours at ambient temperature. The volatiles are removed in vacuo at 40 °C and the residue is purified by silica gel column chromatography using dichloromethane/methanol (40:1). The product is isolated by rotary evaporation at 40 °C and recrystallized from diethyl ether.

Deprotection of the ribose moiety is performed by stirring 2',3'-anisylideneuridine-5'-amide (100 mg) in a mixture of dichloromethane (3 ml), trifluoroacetic acid (0.15 ml) and water (one drop) at ambient temperature. After two hours, the crude product is precipitated by addition of diethyl ether (50 ml), filtered off, dissolved in 7 ml of water : methanol (75 : 25) and purified by RP-HPLC using a gradient of water : methanol from 75 : 25 to water : methanol 0 : 100. Uridine-5'-carboxylic acid[(dibenzylglutamo)carbamoylethyl] amide is isolated by lyophilisation.
Yield over two steps: 170 mg (36 %), white amorphous powder

Uridine-5'-carboxylic acid[(dibenzylglutamo)carbamoylethyl] amide (30 mg, 0.05 mmol) is suspended by sonification in 2 ml of MeOH and water (5:1). Then, the catalyst Pd(OH)₂ (5 mg) is added, the vessel is purged first by argon and then by hydrogen which are applied by means of a balloon. The reaction is performed overnight at ambient temperature and checked by TLC. After 12 hours the catalyst is filtered off and thoroughly washed with methanol and water. The washings are added to the filtrate. The solvent is removed by lyophilisation.
Yield: 20 mg (90 %)
¹H-NMR (500 MHz, D₂O), δ 5.84 (d, 1H, ³J = 6.30 Hz, H-1'), 4.37 (t, 1H, ³J = 6.00 Hz, N-CH (Glu)),4.38 (d, 1H, ³J = 2.50 Hz, H-4'), 4.35 (dd, 1H, ³J = 5.35 Hz and ³J = 6.35 Hz, H-2'), 4.32 (dd, 1H, ³J = 2.20 Hz and ³J = 5.35 Hz, H-3'), 3.74 (m, 2H, ³J = 6.60 Hz, N-CH₂ (dihydrouracil)), 3.52 (t, 2H, ³J = 6.30 Hz, N-CH₂ (carbamoylethylamine)), 2.80 (m, 2H, ³J = 6.30 Hz and ³J = 2.85 Hz, O=C-CH₂ (dihydrouracil)), 2.56 (t, 2H, ³J = 6.30 Hz, O=C-CH₂ (Glu)), 2.45 (t, 2H, ³J = 7.55 Hz, O=C-CH₂ (carbamoylethylamine)), 2.15 (m, 1H, 0.5 x CH₂ (Glu)), 1.96 (m, 1H, 0.5 x CH₂ (Glu)).
¹³C-NMR (500 MHz, D₂O) δ 180.5 (C=O), 179.5 (C=O), 176.6 (C=O), 176.4 (C=O), 174.3 (C-4), 157.6 (C-2), 91.5 (C-1'), 84.8 (C-4'), 75.4 (C-2'), 72.3 (C-3'), 51.7 (N-CH (Glu)), 45.4 (N-CH₂ (dihydrouracil)), 40.6 (O=C-CH₂ (dihydrouracil)), 38.4 (N-CH₂ (carbamoylethylamine) 37.6 (O=C-CH₂ (carbamoylethylamine)), 33.4 (O=C-CH₂ (Glu)), 29.4 (CH₂ (Glu)).

### Example 20: Uridine-5'-carboxylic acid [(1-O-ethylasparto)carbamoylpropyl]-amide

In a dry vessel, N-tert-butyloxycarbonyl-β-alanine (10 mmol, 1890 mg) is dissolved in 10 ml of dry THF and cooled to -25 °C. Subsequently, N-methylmorpholine (10 mmol, 1010 mg) and isobutyl chloroformiate (10 mmol, 1360 mg) are sequentially added under vigorous stirring. Immediately after the formation of a white precipitate (N-methylmorpholine hydrochloride) a solution of racemic aspartic acid diethyl ester hydrochloride (11 mmol, 2475 mg) in 1 M-aq. NaOH-solution (11 ml) is added. The resulting mixture is allowed to warm to ambient temperature. After three hours, THF and other volatiles are removed by rotary evaporation at 40 °C, the residual aqueous mixture is diluted with a small volume of H₂O and adjusted to pH 1 (10% aq. NaHSO₄ solution) and extracted with ethyl acetate (3 x 50 ml). The combined organic layers are washed with satured aq. Na₂CO₃ solution (3 x 20 ml) and subsequently with water (3 x 20 ml), dried over Na₂SO₄, and evaporated to dryness.

The residue (boc-protected amide) is dissolved in 8 ml of dry 4N HCl-dioxane solution and stirred for two hours at ambient temperature. 2-Aminoethylcarboxamido-DL-aspartic acid diethyl ester hydrochloride is precipitated by addition of 50 ml of diethyl ether, filtered off and thoroughly washed with diethyl ether.
Yield over two steps: 2800 mg, 65 %, clay.

Under an atmosphere of argon, 2' ,3' -anisylideneuridine-5' -carboxylic acid (1 mmol, 376 mg), PyBOP^{®} (1.1 mmol, 572 mg) and 1-hydroxybenzotriazole (1.1 mmol, 149 mg) are dissolved in 2 ml of dry DMF at ambient temperature. Diisopropylethylamine (1.1 mmol, 143 mg) and, after one minute, 2-aminoethylcarboxamidoaspartic acid dibenzyl ester hydrochloride (2 mmol, 550 mg), dissolved in a mixture of dry DMF (2 ml) and diisopropylethylamine (0.5 ml), are added sequentially via a syringe to the solution. Vigorous stirring is continued for 24 hours at ambient temperature. The volatiles are removed in vacuo at 40 °C and the residue is purified by silica gel column chromatography using dichloromethane/methanol (40:1). The product is isolated by rotary evaporation at 40 °C and recrystallized from diethyl ether.

Deprotection of the ribose moiety is performed by stirring 2',3'-anisylideneuridine-5'-amide (100 mg) in a mixture of dichloromethane (3 ml), trifluoroacetic acid (0.15 ml) and water (one drop) at ambient temperature. After two hours, the crude product is precipitated by addition of diethyl ether (50 ml), filtered off, dissolved in 7 ml of water : methanol (75 : 25) and purified by RP-HPLC using a gradient of water : methanol from 75 : 25 to water : methanol 0 : 100. Uridine-5'-carboxylic acid [(diethylasparto)carbamoylethyl]amide is isolated by lyophilisation.
Yield over two steps: 250 mg (49 %)

Uridine-5' -carboxylic acid[(diethylasparto)carbamoylethyl] amide is dissolved in 20 ml of aq. Na₂HPO₄-buffered solution (50 mmolar buffer, pH 7.4) at 37°C. Pig liver esterase (20 mg) is added and the slightly cloudy solution is stirred overnight at 37 °C, filtered and lyophilisated. The lyophilisate is dissolved in 7 ml of water/methanol (90 : 10) and purified by RP-HPLC using a gradient of water : methanol from 90 : 10 to water : methanol 0 : 100. The solvents contain 0.1 % of trifuoracetic acid. Uridine-5' -carboxylic acid[(1-O-ethylasparto)carbamoylethyl]amide is isolated by lyophilisation.
Yield: 8 mg (42 %)
¹H-NMR (500 MHz, D₂O), δ 7.97 (d, 1H, ³J = 8.20 Hz, H-6), 5.91 (d, 1H, ³J = 7.90 Hz, H-5), 5.88 (d, 1H, ³J = 5.35 Hz, H-1'), 4.78 (t, 1H, ³J = 6.95 Hz, N-CH (Asp)), 4.51 (pseudo-t, 1H, ³J = 5.35 Hz and ³J = 5.05 Hz, H-2'), 4.46 (d, 1H, ³J = 4.70 Hz, H-4'), 4.42 (pseudo-t, 1H, ³J = 5.00 Hz and ³J = 4.75 Hz, H-3'), 4.18 (q, 2H, O-CH₂), 3.28 (dt, 2H, ³J = 6.60 Hz and ³J = 6.95 Hz, N-CH₂ (carbamoylpropylamine)), 2.91 (AB-system with A dd and B dd, 2H, ³J = 5.35 Hz and ²J = 16.70 Hz, O=C-CH₂ (Asp)), 2.34 (t, 2H, ³J = 6.95 Hz, O=C-CH₂ (carbamoylpropylamine)), 1.83 (dt, 2H, ³J = 7.25 Hz and ³J = 6.95 Hz, CH₂ (carbamoylpropylamine)), 1.27 (t, 3H, CH₃).
¹³C-NMR (500 MHz, D₂O), δ 178.6 (C=O), 176.8 (C=O), 175.4 (C=O), 174.2 (C=O), 169.0 (C-4), 154.5 (C-2), 146.1 (C-6), 105.2 (C-5), 94.3 (C-1'), 85.6 (C-4'), 73.4 (C-2'), 73.1 (C-3'), 65.1 (O-CH₂), 51.9 (N-CH (Asp)), 41.3 (N-CH₂ (carbamoylpropylamine)), 38.8 (O=C-CH₂ (Asp)), 35.5 (O=C-CH₂ (carbamoylpropylamine)), 27.4 (CH₂ (carbamoylpropylamine)), 16.1 (CH₃).

### Example 21: NTPDase assays by capillary electrophoresis (CE)

The applied enzyme inhibition assay has been described (Iqbal, J. et al., Purinergic Signalling 2005, 1: 349-358).

### (A) CE instrumentation

All experiments were carried out using a P/ACE MDQ capillary electrophoresis system (Beckman Instruments, Fullerton, CA, USA) equipped with a UV detection system coupled with a diode-array detector (DAD). Data collection and peak area analysis were performed by the P/ACE MDQ software 32 KARAT obtained from Beckman Coulter. The capillary temperature was kept constant at 25 °C. The temperature of the sample storing unit was also adjusted to 25 °C. The electrophoretic separations were carried out using an eCAP polyacrylamide-coated fused-silica capillary (30 cm (20 cm effective length) x 50 µm internal diameter (I.D.) x 360 µm outside diameter (O.D.), obtained from CS-Chromatographie (Langerwehe, Germany)). The separation was performed using an applied current of -60 pA and a data acquisition rate of 8 Hz. Analytes were detected using direct UV absorbance at 210 nm. The capillary was conditioned by rinsing with water for 2 min and subsequently with buffer (phosphate 50 mM, pH 6.5) for 1 min. Sample injections were made at the cathodic side of the capillary.

### (B) NTPDase inhibition assay by capillary electrophoresis

Enzyme inhibition assays were carried out at 37 °C in a final volume of 100 µl. The reaction mixture contained 320 µM of ATP (substrate) in reaction buffer. The reaction buffer contained 140 mM NaCl, 5 mM KCl, 1 mM MgCl₂, 2 mM CaCl₂, and 10 mM Hepes, pH 7.4.

Different concentrations of test compounds dissolved in DMSO or water (10 µl) were added and the reaction was initiated by the addition of 10 µl of the appropriately diluted recombinant or native human or rat NTPDase enzyme. The final DMSO concentration did not exceed 1 %. The mixture (final volume 100 µl) was incubated for 15 min and terminated by heating at 99 °C for 4 min. Aliquots of the reaction mixture (50 µl) were then transferred to mini-CE vials and injected into the CE instrument under the conditions described above. 20 µM of UMP was used as internal standard. Inhibition of NTPDase was tested over a range of 6 to 8 concentrations of test compound spanning 3 orders of magnitude to determine Kᵢ values. Each analysis was repeated two times (in triplicates) in two separate experiments. The Cheng-Prusoff equation was used to calculate the Kᵢ values from the IC₅₀ values, determined by the non-linear curve fitting program PRISM^{®} 3.0 (GraphPad, San Diego, California, USA). In table 1 Kᵢ values of selected compounds of examples 1 to 20 as inhibitors of human NTPDases (hNTPDases) are given.

**Table 1: Kᵢ values for human NTPDase inhibition obtained for selected compounds using the capillary electrophoresis (CE) method. The results are means ± SEM of three separate experiments each run in triplicate. Generally a compound with an Ki value of more than 500 µM is considered as inactive.**

| Example No. | Structure | hNTPDase1 Kᵢ [µM] ± SEM | hNTPDase2 Kᵢ [µM] ± SEM |
|---|---|---|---|
| 1 | 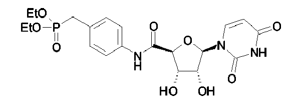 | | 117 ± 15 |
| 2 | 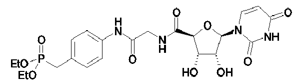 | | 8.2 ± 2.1 |
| 8 | 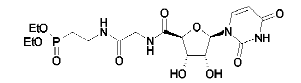 | | 116 ± 24 |
| 6 | 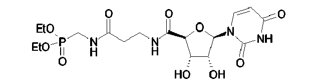 | | 167 ± 21 |
| 3 | 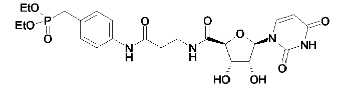 | | 173 ± 17 |
| 7 | 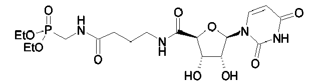 | | 29.2 ± 2.7 |
| 12 | 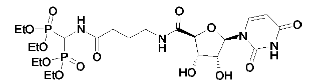 | 32.4 ± 1.1 | |
| 11 | 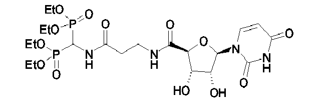 | 325 ± 25 | |
| 10 | 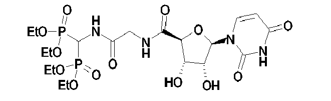 | 93 ± 14 | |
| 13 | 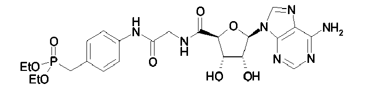 | | inactive |
| 15 | 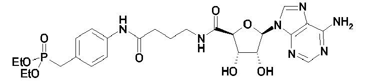 | 161 ± 24 | 213 ± 34 |
| 20 | 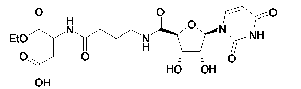 | 29 ± 4 | |
| 18 | 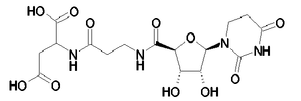 | 55 ± 5 | |
| 19 | 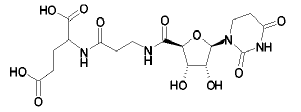 | 10.8 ± 1 | |

In table 2 inhibition data of selected examples at human P2Y receptor subtypes (hP2Y₂, hP2Y₄ and hP2Y₆ are uracil nucleotide sensitive receptors) are shown. It can be seen that no compound was identified that inhibited P2Y receptors at high concentrations of 100 µM or higher.

**Table 2: Percent inhibition values at selected P2Y receptor subtypes obtained for selected compounds. The results are means ± SEM of three separate experiments each run in triplicate.**

| Example No. | Structure | hP2Y₂ percent inhibition of UTP binding at 100 µM | hP2Y₄ percent inhibition of UTP binding at 100 µM | hP2Y₆ percent inhibition of UDP binding at 100 µM | hP2Y₁₂ percent Inhibition of [³H]PSB-0413 Binding at 10µM |
|---|---|---|---|---|---|
| 1 | 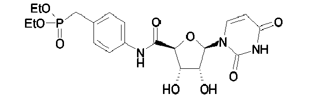 | | | -12 ± 3 | |
| 2 | 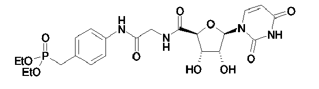 | | | -10 ± 10 | |
| 8 | 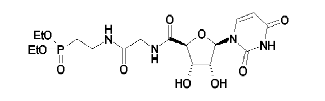 | | | 9 ± 13 | |
| 6 | 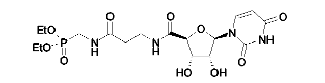 | | | - 3 ± 2 | |
| 3 | 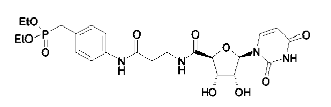 | | | 14 ± 6 | |
| 12 | 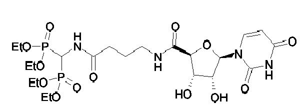 | -44 ± 20 | 27 ± 7 | | 1 ± 5 |
| 11 | 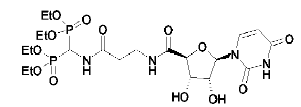 | -12 ± 24 | 7 ± 20 | | 1 ± 5 |
| 10 | 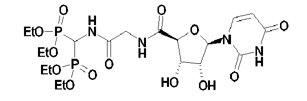 | -38 ± 11 | 34 ± 15 | | 2 ± 5 |
| 13 | 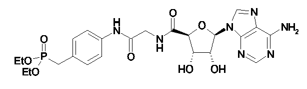 | | | -8 ± 11 | |
| 20 | 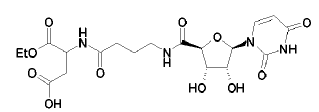 | 6 ± 17 | 2 ± 9 | | |
| 18 | 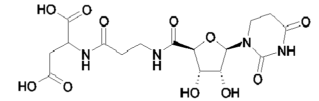 | | | | 2 ± 5 % |
| 19 | 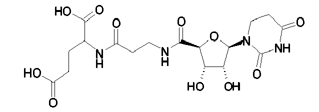 | | | | 6 ± 4 % |

In tables 3 and 4 data for standard NTPDase inhibitors are shown. It can be seen that - with the exception of the ATP analog ARL 67156 - the compounds are non-selective inhibitors of NTPDase1, 2 and 3, and they are at least equally potent as antagonists at one or several P2 receptor subtypes.

**Table 3: Kᵢ values at rat NTPDase1, 2 and 3 and at selected P2Y receptor subtypes obtained for standard compounds: reactive blue 2 (RB2), PPADS, suramin, and ARL67156, using the in-capillary electrophoresis method. The results are means ± SEM of three separate experiments each run in duplicate. A compound with an Ki value of more than 500 µM is considered as inactive.**

| Inhibitor | Kᵢ ± SEM [µM] | | | | | | |
|---|---|---|---|---|---|---|---|
| | Ectonucleotidases | | | selected P2Y-Receptors | | | |
| | NTPDase1 | NTPDase 2 | NTPDase 3 | P2Y₂ | P2Y₄ | P2Y₆ | P2Y₁₂ |
| RB2 | 20.0 ± 0.003 | 24.2 ± 0.06 | 1.10 ± 0.03 | 1** | > 100** | 31** | 1.3*** |
| PPADS | 46.0 ± 0.01 | 44.2 ± 0.03 | 3.0 ± 0.001 | inactive** | 73% inhibition at 100 µM¹² | 69% inhibition at 100 µM¹² | |
| Suramin | 300 ± 0.1 | 65.4 ± 0.01 | 12.7 ± 0.03 | 50** | inactive* * | > 100** | 4.0*** |
| ARL 67156 | 27.0 ± 0.004 | ≥ 1,000* | 112.1 ± 0.05 | | | | |

| | | | | | | | |
|---|---|---|---|---|---|---|---|
| * 50 % inhibition at 1 mM concentration ** Brunschweiger, A. and Müller, C.E., Curr. Med. Chem. 2006; 13: 289-312 *** King, B.F. and Townsend-Nicholson, A., Scientific review on TOCRIS | | | | | | | |

**Table 4: Kᵢ values at rat NTPDase1, 2 and 3 and at selected P2X receptor subtypes**

| Inhibitor | Kᵢ ± SEM [µM] | | | | | | |
|---|---|---|---|---|---|---|---|
| | Ectonucleotidases | | | selected P2X-Receptors | | | |
| | NTPDase1 | NTPDase 2 | NTPDase 3 | P2X₁ | P2X₂ | P2X₃ | P2X₅ |
| RB2 | 20.0 ± 0.003 | 24.2 ± 0.06 | 1.10 ± 0.03 | 2*** | 0.4*** | 50*** | 20*** |
| PPADS | 46.0 ± 0.01 | 44.2 ± 0.03 | 3.0 ± 0.001 | 0.13** * | 1. 6*** | 0. 2*** | 0. 2*** |
| Suramin | 300 ± 0.1 | 65.4 ± 0.01 | 12.7 ± 0.03 | 2*** | 10*** | 4*** | 1.6*** |
| ARL 67156 | 27.0 ± 0.004 | ≥ 1,000* | 112.1 ± 0.05 | | | | |

| | | | | | | | |
|---|---|---|---|---|---|---|---|
| * 50 % inhibition at 1 mM concentration *** King, B.F. and Townsend-Nicholson, A., Scientific review on TOCRIS | | | | | | | |

## Claims

1. A compound represented by the formula 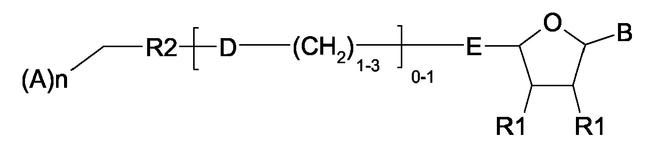 wherein
D represents a moiety selected from the group consisting of a single bond, -O-, -S-, -CH₂-, -CHR3-, -NH-, -NR3-, -CO-, -CH₂CO-, 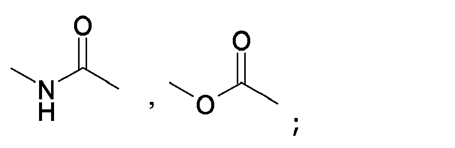
E represents a moiety selected from the group consisting of -R5-, -O-R5-, -SCH₂- and-NH-R5-;
B represents a purinyl or pyrimidinyl residue which is connected with the furanoside ring via one of its nitrogen atoms;
R1 represent independently from each other residues selected from the group consisting of hydroxyl, hydrogen, C₁-C₃-alkoxyl, C₁-C₃-alkyl, C₁-C₃-alkenyl, C₁-C₃-alkinyl, C₁-C₃-acyl, halogen, or commonly form a double bond with one of the vicinal C atoms or an acetyl or ketal ring with each other;
R2 is -(CH₂)₀₋₂- or phenyliden;
n is 1 or 2;
A represents a -PO(OR3)₂, -SO2(OR3), or -(CH₂)ₘ-COOR4 residue, wherein m is an integer from 0 to 2, R3 is a C₁-C₃-alkyl and R4 is selected from the group consisting of hydrogen and C₁-C₃-alkyl; and
R5 is a carbonyl or methylidene group.

2. The compound of claim 1, which is represented by the formula 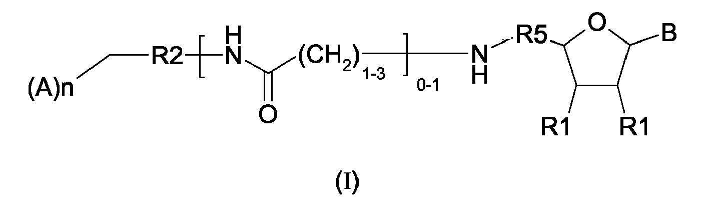 wherein
B represents a purinyl or pyrimidinyl residue which is connected with the furanoside ring via one of its nitrogen atoms;
R1 represent independently from each other residues selected from the group consisting of hydroxyl, hydrogen, C₁-C₃-alkoxyl, C₁-C₃-alkyl, C₁-C₃-alkenyl, C₁-C₃-alkinyl, C₁-C₃-acyl, halogen, or commonly form a double bond with one of the vicinal C atoms or an acetyl or ketal ring with each other;
R2 is -(CH₂)₀₋₂- or phenyliden;
n is 1 or 2;
A represents a -PO(OR3)₂, -SO2(OR3), or -(CH₂)ₘ-COOR4 residue, wherein m is an integer from 0 to 2, R3 is a C₁-C₃-alkyl and R4 is selected from the group consisting of hydrogen and C₁-C₃-alkyl; and
R5 is a carbonyl or methylidene group.

3. The compound of claim 2, which is represented by the formula 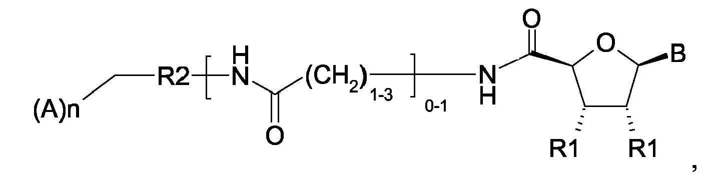 wherein all variables are defined as in claim 2.

4. The compound of any one of claims 1 to 3, wherein
at least one R1 is OH and the other R1 is H or OH; and/or
B is selected from the group consisting of uracilyl, adeninyl or a derivative thereof, preferably is 1-uracilyl or 9-adeninyl, more preferably is 1-uracilyl.

5. The compound of any one of claims 1 to 4, wherein
A represents a -PO(OR3)₂ residue; and wherein
R3 is ethyl and/or n is 1.

6. The compound of claim 5, which is represented by the formula 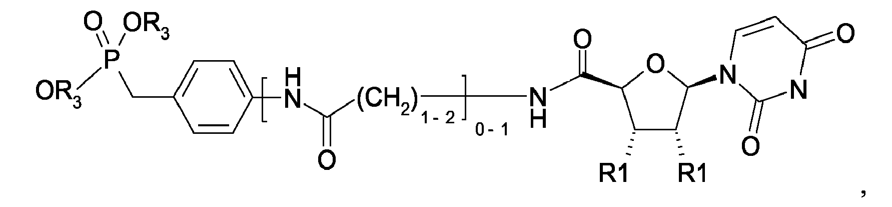 and wherein preferably
(i) at least one R1 is OH and the other R1 is H or OH, more preferably both R1 are OH; and/or
(ii) R3 is ethyl.

7. The compound of claim 6, which is represented by the formula

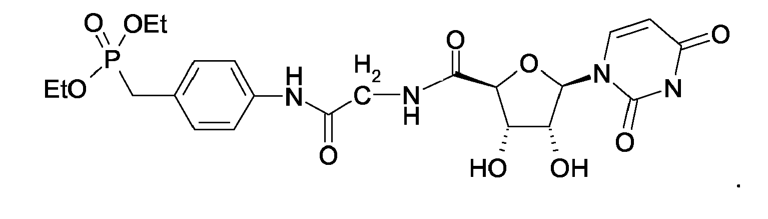

8. The compound of any one of claims 1 to 4, wherein
A represents a -(CH₂)ₘ-COOR4 residue; and wherein
R4 is H and/or n is 2.

9. The compound of claim 8, which is represented by the formula

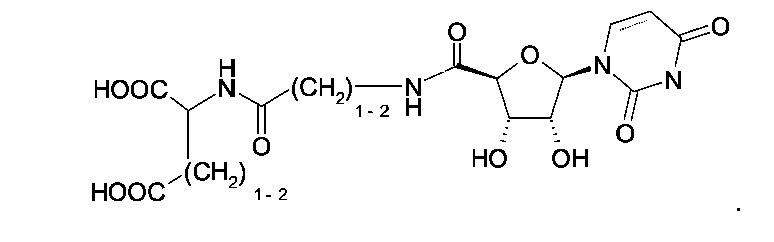

10. A pharmaceutical or diagnostic composition comprising a compound as defined in any one of claims 1 to 9.

11. Use of the compound of any one of claims 1 to 9 for the preparation of a medicament for treating diseases connected with a reduced abundance of nucleotides in a patient or for therapies aiming at increasing the nucleotide concentration in a patient.

12. The use of claim 11, wherein the medicament is for therapy of dry eye disease, respiratory diseases, cystic fibrosis, inflammatory diseases, diseases of the immune system, gastrointestinal diseases, kidney disorders, cancer, and brain diseases.

13. Use of the compound of any of claims 1 to 9 as selective NTPDase inhibitor.

14. An *in vitro* method for ATP quantification using the compound of any of claims 1 to 9, preferably a luciferase assay.

15. A method for preparing the compound of formula (I) of any one of claims 2 to 9, which preferably comprises reacting a compound of formula (II) 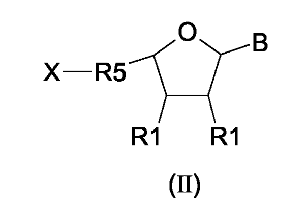 wherein X is a leaving group and all other variables are as defined in claim 2, with a compound of formula (III) 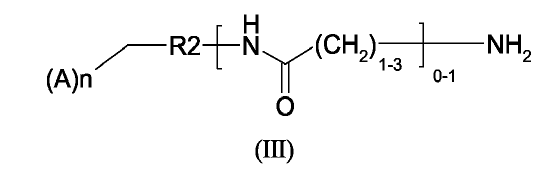 wherein all variables are as defined in claim 2.
